(19) Europäisches Patentamt
European Patent Office
Office européen des brevets

(11) Publication number: **0 221 025**
**A1**

# (12) EUROPEAN PATENT APPLICATION

(21) Application number: **86810470.4**

(22) Date of filing: **21.10.86**

(51) Int. Cl.[4]: **C 07 D 207/337**
C 07 D 307/54,
C 07 D 333/24, A 61 K 31/34,
A 61 K 31/38, A 61 K 31/40

(30) Priority: **25.10.85 US 791198 07.01.86 US 816664**

(43) Date of publication of application:
**06.05.87 Bulletin 87/19**

(84) Designated Contracting States:
**AT BE CH DE ES FR GB GR IT LI LU NL SE**

(71) Applicant: **SANDOZ AG**
**Lichtstrasse 35**
**CH-4002 Basel (CH)**

(84) Designated Contracting States:
**BE CH ES FR GB GR IT LI LU NL SE**

(71) Applicant: **SANDOZ-PATENT-GMBH**
**Humboldtstrasse 3**
**D-7850 Lörrach (DE)**

(84) Designated Contracting States: **DE**

(71) Applicant: **SANDOZ-ERFINDUNGEN**
**Verwaltungsgesellschaft m.b.H.**
**Brunner Strasse 59**
**A-1235 Wien (AT)**

(84) Designated Contracting States: **AT**

(72) Inventor: **Wareing, James Richard**
**402 Millbrook Avenue**
**Randolph, N.J. 07801 (US)**

**Damon, Robert Edson**
**23156 West View**
**Wharton, N.J. 07885 (US)**

## (54) Heterocyclic analogs of mevalonolactone and derivatives thereof, processes for their production and their use as pharmaceuticals.

(57) Compounds of formula

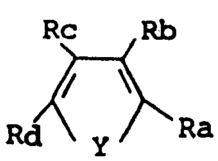

I

wherein
Ra is a group -X-Z, Rb is $R_2$, Rc is $R_3$, Rd is $R_4$ and Y is a group
-N- or
|
$R_1$
Ra is $R_1$, Rb is a group -X-Z, Rc is $R_2$, Rd is $R_3$ and Y is O, S or a group -N-;
|
$R_4$
$R_1$, $R_2$, $R_3$, and $R_4$ independently are $C_{1-6}$alkyl not containing an asymmetric carbon atom, $C_{3-7}$cycloalkyl or a ring

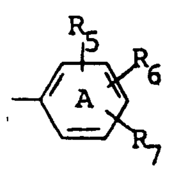

or in the case of $R_3$ and $R_4$ additionally hydrogen, or for $R_3$ when Y is O or S

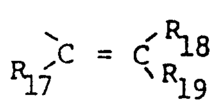

whereby $R_{17}$ is hydrogen or $C_{1-3}$alkyl and $R_{18}$ and $R_{19}$ are independently hydrogen $C_{1-3}$alkyl or phenyl; each $R_5$ is independently hydrogen, $C_{1-3}$alkyl, n-butyl, i-butyl, t-butyl, $C_{1-3}$alkoxy, n-butoxy, i-butoxy, trifluoromethyl, fluoro, chloro, bromo, phenyl, phenoxy or benzyloxy; each $R_6$ is independently hydrogen, $C_{1-3}$alkyl, $C_{1-3}$alkoxy, trifluoromethyl, fluoro, chloro, bromo, phenoxy or benzyloxy, and each $R_7$ is independently hydrogen, $C_{1-2}$alkyl, $C_{1-2}$alkoxy, fluoro or chloro.

EP 0 221 025 A1

with the proviso that there may only be one each of trifluoromethyl, phenoxy or benzyloxy in each ring A present. X is $(CH_2)_m$ or $(CH_2)_qCH=CH-(CH_2)_q$. m is 0, 1, 2 or 3 and both q's are 0 or one is 0 and the other is 1.

Z is 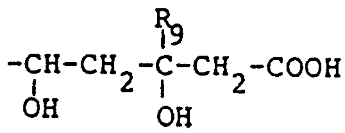 II

wherein $R_9$ is hydrogen or $C_{1-3}$alkyl.
in free acid form, or in the form of an ester or δ-lactone thereof or in salt form as appropriate, which compounds are indicated for use as pharmaceuticals in particular as hypolipoproteinemic and anti-atherosclerotic agents.

**Description**

HETEROCYCLIC ANALOGS OF MEVALONOLACTONE AND DERIVATIVES THEREOF, PROCESSES FOR THEIR PRODUCTION AND THEIR USE AS PHARMACEUTICALS

The present invention concerns heterocyclic analogs of mevalonolactone and derivatives thereof, processes for their preparation, pharmaceutical compositions containing them and their use as pharmaceuticals especially as agents for treating hyper-lipoproteinemia and atherosclerosis.

More particularly the invention concerns compounds of formula I

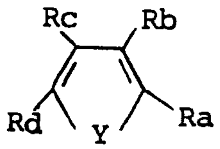

I

wherein

Ra is a group -X-Z, Rb is $R_2$, Rc is $R_3$, Rd is $R_4$ and Y is a group 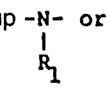 or

Ra is $R_1$, Rb is a group -X-Z, Rc is $R_2$, Rd is $R_3$ and Y is O, S or a group 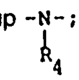;

$R_1$, $R_2$, $R_3$ and $R_4$ independently are $C_{1-6}$alkyl not containing an asymmetric carbon atom, $C_{3-7}$cycloalkyl or a ring

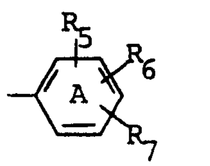

or in the case of $R_3$ and $R_4$ additionally hydrogen, or for $R_3$ when Y is O or S

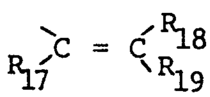

whereby $R_{17}$ is hydrogen or $C_{1-3}$alkyl and $R_{18}$ and $R_{19}$ are independently hydrogen $C_{1-3}$alkyl or phenyl; each $R_5$ is independently hydrogen, $C_{1-3}$alkyl, n-butyl, i-butyl, t-butyl, $C_{1-3}$alkoxy, n-butoxy, i-butoxy, trifluoromethyl, fluoro, chloro, bromo, phenyl, phenoxy or benzyloxy; each $R_6$ is independently hydrogen, $C_{1-3}$alkyl, $C_{1-3}$alkoxy, trifluoromethyl, fluoro, chloro, bromo, phenoxy or benzyloxy, and each $R_7$ is independently hydrogen, $C_{1-2}$alkyl, $C_{1-2}$alkoxy, fluoro or chloro, with the proviso that there may only be one each of trifluoromethyl, phenoxy or benzyloxy in each ring A present, X is $(CH_2)_m$ or $(CH_2)_qCH=CH-(CH_2)_q$, m is 0, 1, 2 or 3 and both q's are 0 or one is 0 and the other is 1,

Z is 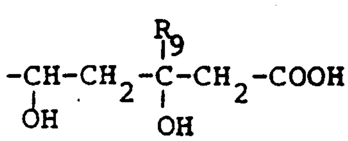 II

wherein $R_9$ is hydrogen or $C_{1-3}$alkyl,
in free acid form, or in the form of an ester or δ-lactone thereof or in salt form as appropriate.

Suitable esters include physiologically acceptable esters e.g. physiologically hydrolysable and -acceptable esters.

By the term "physiologically-hydrolysable and -acceptable ester is meant an ester of a compound in accordance with the invention in which the carboxyl moiety if present is esterified, and which is hydrolysable under physiological conditions to yield an alcohol which is itself physiologically acceptable, e.g. non-toxic at desired dosage levels. For the avoidance of doubt, throughout this specification it is the right hand side of the X radical that is attached to the Z group. Preferred such acids, esters and salt forms as Z can be represented together with the free acid by formula a

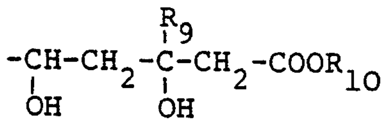

a

wherein

$R_9$ is hydrogen or $C_{1-3}$alkyl and

$R_{10}$ is hydrogen, a physiologically acceptable ester forming group ($R_{11}$) or a pharmaceutically acceptable cation (M).

When Z is in lactone form it forms a δ-lactone of formula b

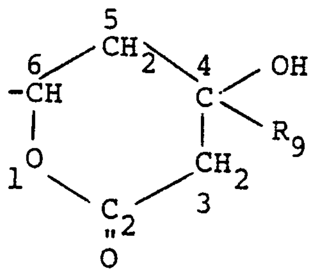

b

and reference to "lactone" hereinafter refer to δ-lactones.

Salts of the compounds of the invention, e.g. of the compounds of formula I, include in particular their pharmaceutically acceptable salts. Such pharmaceutically acceptable salts include e.g. alkali metal salts such as the sodium and potassium salts and salts with ammonium.

References to compounds of formulae I and IA-ID and subscopes thereof are intended to cover all forms unless otherwise stated.

Depending on the nature of the various substituents the compounds of formula I may be divided into four main groups, namely

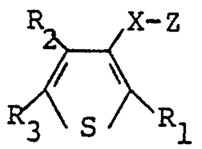

IA ,

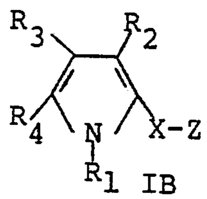

IB ,

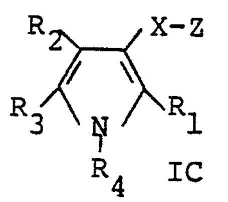

IC ,

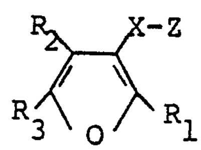

ID

These four groups may be further divided into two sub-groups each depending on the significance of Z as either a group of formula II in other than lactone form (sub-group "a") or a group of formula b (sub-group "b"). The resulting eight sub-groups are designated as formulae IAa, IAb, IBa, IBb, ICa, ICb, IDa, IDb respectively.

As is self-evident to those skilled in the art, each compound of formula I (and every sub-group and species thereof) has at least two centres of asymmetry (e.g. the two carbon atoms bearing the hydroxy groups in the group of formula a and the carbon atom bearing the hydroxy group and the carbon atom having the free valence in the group of formula b) and these lead (e.g. with two centres) to four stereoisomic forms (enantiomers) of each compound (two racemates or pairs of diasteroisomers). In preferred compounds having only two such centres of asymmetry these four stereoisomers may be designated as the R,R; R,S; S,R; and S,S enantiomers, all four stereoisomers being within the scope of this invention. Depending on the nature of substituents further asymmetric carbon atoms may be present and the resulting isomers and mixtures thereof also form part of the invention. Compounds containing only two centres of asymmetry (four menmentioned stereoisomers) are preferred.

Preferably in compounds IA-ID one of $R_1$ and $R_2$ is $C_{1-6}$alkyl not containing an asymmetric carbon atom and the other is a Ring A. Also preferably in compounds IB and IC, one of $R_3$ and $R_4$ is a Ring A and the other is hydrogen or $C_{1-6}$alkyl not containing an asymmetric carbon atom, preferably hydrogen or $C_{1-2}$alkyl and most preferably hydrogen except that $R_4$ in compounds IC is preferably other than hydrogen. More preferably, the preferences of both preceding sentences occur simultaneously. Thus, the preferred compounds IB and IC and each of the sub-scopes thereof are those having attached to the pyrrole ring two Rings A and two alkyl groups or in compounds IB especially one alkyl group and one hydrogen. Even more preferably the two Rings A are ortho to each other. Also preferably the pyrrole ring does not bear two ortho tertiary alkyl groups.

In Formula IB:

$R_1$ is preferably $R_{1Bx}$, where $R_{1Bx}$ is Ring A, more preferably $R_1'{}_{Bx}$, where $R_1'{}_{Bx}$ is Ring A wherein $R_5$ is $R_5'$, $R_6$ is $R_6'$, and $R_7$ is $R_7'$, even more preferably $R_1''{}_{Bx}$, where $R_1''{}_{Bx}$ is Ring A wherein $R_5$ is $R_5''$, $R_6$ is $R_6''$, and $R_7$ is hydrogen, and most preferably phenyl, 4-fluorophenyl or 3,5-dimethylphenyl, especially

4-fluorophenyl; or

$R_1$ is preferably $R_{1By}$, where $R_{1By}$ is $C_{1-6}$alkyl not containing an asymmetric carbon atom, more preferably $R'_{1By}$, where $R'_{1By}$ is $C_{1-4}$alkyl not containing an asymmetric carbon atom, and most preferably i-propyl.

$R_2$ is preferably $R_{2Bx}$, where $R_{2Bx}$ is $C_{1-6}$alkyl not containing an asymmetric carbon atom, more preferably $R'_{2Bx}$, where $R'_{2Bx}$ is $C_{1-4}$alkyl not containing an asymmetric carbon atom, and most preferably i-propyl, or

$R_2$ is preferably $R_{2By}$, where $R_{2By}$ is Ring A, more preferably $R'_{2By}$, where $R'_{2By}$ is Ring A wherein $R_5$ is $R'_5$, $R_6$ is $R'_6$, and $R_7$ is $R'_7$, even more preferably $R''_{2By}$, where $R''_{2By}$ is Ring A wherein $R_5$ is $R''_5$, $R_6$ is $R''_6$ and $R_7$ is hydrogen, and most preferably phenyl, 4-fluorophenyl or 3,5-dimethylphenyl, especially 4-fluorophenyl.

$R_3$ is preferably $R_{3Bx}$, where $R_{3Bx}$ is hydrogen or $C_{1-6}$alkyl not containing an asymmetric carbon atom, more preferably $R'_{3Bx}$, where $R'_{3Bx}$ is hydrogen or $C_{1-2}$alkyl, even more preferably $R''_{3Bx}$, where $R''_{3Bx}$ is hydrogen or methyl, and most preferably hydrogen; or

$R_3$ is preferably $R_{3By}$, where $R_{3By}$ is Ring A, more preferably $R'_{3By}$, where $R'_{3By}$ is Ring A wherein R is $R'_5$, $R_6$ is $R'_6$, and $R_7$ is $R'_7$, even more preferably $R''_{3By}$, where $R''_{3By}$ is Ring A wherein $R_5$ is $R''_5$, $R_6$ is $R''_6$, and $R_7$ is hydrogen, and most preferably phenyl.

$R_4$ is preferably $R_{4Bx}$, where $R_{4Bx}$ is Ring A, more preferably $R'_{4Bx}$ where $R'_{4Bx}$ is Ring A wherein $R_5$ is $R'_5$, $R_6$ is $R'_6$, and $R_7$ is $R'_7$, even more preferably $R''_{4Bx}$, where $R''_{4Bx}$ is Ring A wherein $R_5$ is $R''_5$, $R_6$ is $R''_6$, and $R_7$ is hydrogen, and most preferably phenyl; or $R_4$ is preferably $R_{4By}$, where $R_{4By}$ is hydrogen or $C_{1-6}$alkyl not containing an asymmetric carbon atom, more preferably $R'_{4By}$, where $R'_{4By}$ is hydrogen or $C_{1-2}$alkyl, even more preferably $R''_{4By}$, where $R''_{4By}$ is hydrogen or methyl, and most preferably hydrogen.

In Formulae IA, IC and ID:

$R_1$ is preferably $R_{1Cx}$, where $R_{1Cx}$ is $C_{1-6}$alkyl not containing an asymmetric carbon atom, more preferably $R'_{1Cx}$, where $R'_{1Cx}$ is $C_{1-4}$alkyl not containing an asymmetric carbon atom, and most preferably i-propyl, or

$R_1$ is preferably $R_{1Cy}$, where $R_{1Cy}$ is Ring A, more preferably $R'_{1Cy}$, where $R'_{1Cy}$ is Ring A wherein $R_5$ is $R'_5$, $R_6$ is $R'_6$ and $R_7$ is $R'_7$, even more preferably $R''_{1Cy}$, where $R''_{1Cy}$ is Ring A wherein $R_5$ is $R''_5$, $R_6$ is $R''_6$, and $R_7$ is hydrogen, and most preferably phenyl, 4-fluorophenyl or 3,5-dimethylphenyl, especially 4-fluorophenyl.

$R_2$ is preferably $R_{2Cx}$, where $R_{2Cx}$ is Ring A, more preferably $R'_{2Cx}$, where $R'_{2Cx}$ is Ring A wherein $R_5$ is $R'_5$, $R_6$ is $R'_6$ and $R_7$ is $R'_7$, even more preferably $R''_{2Cx}$, where $R''_{2Cx}$ is Ring A wherein $R_5$ is $R''_5$, $R_6$ is $R''_6$, and $R_7$ is hydrogen, and most preferably phenyl, 4-fluorophenyl or 3,5-dimethylphenyl, especially 4-fluorophenyl; or

$R_2$ is preferably $R_{2Cy}$, where $R_{2Cy}$ is $C_{1-6}$alkyl not containing an asymmetric carbon atom, more preferably $R'_{2Cy}$, where $R'_{2Cy}$ is $C_{1-4}$alkyl not containing an asymmetric carbon atom, and most preferably i-propyl.

$R_3$ is preferably $R_{3Cx}$, where $R_{3Cx}$ is Ring A, more preferably $R'_{3Cx}$, where $R'_{3Cx}$ is Ring A wherein $R_5$ is $R'_5$, $R_6$ is $R'_6$, and $R_7$ is $R'_7$, even more preferably $R''_{3Cx}$, where $R''_{3Cx}$ is Ring A wherein $R_5$ is $R''_5$, $R_6$ is $R''_6$, and $R_7$ is hydrogen, and most preferably phenyl; or

$R_3$ is preferably $R_{3Cy}$, where $R_{3Cy}$ is hydrogen or $C_{1-6}$alkyl not containing an asymmetric carbon atom, more preferably $R'_{3Cy}$, where $R'_{3Cy}$ is hydrogen or $C_{1-2}$alkyl, and even more preferably $R''_{3Cy}$, where $R''_{3Cy}$ is hydrogen or methyl, especially hydrogen.

In the compounds of formulae IA and ID, especially the former, $R_{3cy}$, $R_{3cy}'$ and $R_{3cy}''$ include $-CH=C(CH_3)_2$.

In formula IC:

$R_4$ is preferably $R_{4Cx}$, where $R_{4Cx}$ is hydrogen or $C_{1-6}$alkyl not containing an asymmetric carbon atom, more preferably $R'_{4Cx}$, where $R'_{4Cx}$ $C_{1-2}$alkyl, even more preferably methyl, or

$R_4$ is preferably $R_{4Cy}$, where $R_{4Cy}$ is Ring A, more preferably $R'_{4Cy}$, where $R'_{4Cy}$ is Ring A wherein R is $R'_5$, $R_6$ is $R'_6$, and $R_7$ is $R'_7$, even more preferably $R''_{4Cy}$, where $R''_{4Cy}$ is Ring A wherein $R_5$ is $R''_5$, $R_6$ is $R''_6$, and $R_7$ is hydrogen, and most preferably phenyl.

In addition, in the compounds IA and ID $R_2$ is preferably $C_{1-6}$alkyl not containing an asymmetric carbon atom, especially isopropyl or t-butyl, or phenyl or p-substituted phenyl, especially p-fluorophenyl and $R_1$ is preferably phenyl or p-substituted phenyl especially p-fluorophenyl.

Of IA and ID the former are preferred.

In each of IA, IB, IC and ID the following preferences apply.

Each $R_5$ independently is preferably $R_5'$ where $R_5'$ is hydrogen, $C_{1-3}$alkyl, $C_{1-2}$alkoxy, trifluoromethyl, fluoro or chloro, more preferably $R_5''$ where $R_5''$ is hydrogen methyl or fluoro. In the case of $R_1$ and $R_2$ being a Ring A each $R''_5$ is preferably fluoro, especially 4-fluoro. In the case of $R_3$ and $R_4$ being a Ring A $R_5''$ is preferably hydrogen.

Each $R_6$ independently is preferably $R_6'$ where $R_6'$ is hydrogen, $C_{1-2}$alkyl, fluoro or chloro more preferably $R_6''$ where $R_6''$ is hydrogen or methyl, most preferably hydrogen.

Each $R_7$ independently is preferably $R_7'$ where $R_7'$ is hydrogen or methyl, most preferably hydrogen.

Preferably, each Ring A, independently bears a maximum of one substituent selected from the group consisting of t-butyl, trifluoromethyl, phenyl, phenoxy and benzyloxy. More preferably, when any two or all three of the substituents on each Ring A independently are ortho to each other, at least one member of each pair that are ortho to each other is a member of the group consisting of hydrogen, methyl, methoxy, fluoro and

chloro. Also more preferably, at least one of the ortho positions of each Ring A, independently has a member of the group consisting of hydrogen, fluoro and methyl.

When $R_1$ and/or $R_2$ is a Ring A these are independently preferably phenyl, 4-fluorophenyl or 3,5-dimethylphenyl, more preferably the latter two and most preferably 4-fluorophenyl.

When $R_3$ and/or $R_4$ is a Ring A these are independently preferably phenyl.

$R_9$ is preferably $R_9'$, where $R_9'$ is hydrogen or methyl, and most preferably hydrogen and $R_{10}$ is preferably $R_{10}'$, where $R_{10}'$ is hydrogen, $R_{11}'$ or M, more preferably $R_{10}''$, where $R_{10}''$ is hydrogen, $C_{1-3}$alkyl or M, even more preferably $R_{10}'''$, where $R_{10}'''$ is hydrogen, $C_{1-2}$alkyl or M, and most preferably M, particularly M' and especially sodium.

$R_{11}$ is preferably $R_{11}'$, where $R_{11}'$ is $C_{1-3}$alkyl, n-butyl, i-butyl, t-butyl or benzyl, more preferably $R_{11}''$, where $R_{11}''$ is $C_{1-3}$alkyl, and most preferably $R_{11}'''$, where $R_{11}'''$ is $C_{1-2}$alkyl, especially ethyl.

Any -CH=CH-, -CH=CH-$CH_2$- or -$CH_2$-CH=CH- as X is preferably trans, i.e., (E).

X is preferably X', where X' is -$CH_2CH_2$- or -CH=CH-, and most preferably

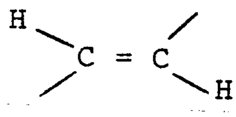

(i.e., (E)-CH=CH-).

Z is preferably a group of formula a wherein $R_9$ is $R_9'$ (especially hydrogen), and $R_{10}$, $R_{10}'$ or a group of formula b wherein $R_9$ is $R_9'$ (especially hydrogen), more preferably a group of formula a wherein $R_9$ is hydrogen, and $R_{10}$ is $R_{10}''$ or a group of formula b wherein $R_9$ is hydrogen, even more preferably a group of formula a wherein $R_9$ is hydrogen, and $R_{10}$ is $R_{10}'''$ or a group of formula b wherein $R_9$ is hydrogen, and most preferably a group of formula a wherein $R_9$ is hydrogen, and $R_{10}$ is M, preferably M' and especially sodium.

m is preferably m', where m' is 2 or 3, most preferably 2.

M is preferably free from centers of asymmetry and is more preferably M', i.e., sodium, potassium or ammonium, and most preferably sodium. For simplicity, each formula in which M appears has been written as if M were monovalent and, preferably, it is. However, it may also be divalent or trivalent and, when it is, it balances the charge of two or three carboxy groups, respectively. Thus, formula I and every other formula containing an M embraces compounds wherein M is divalent or trivalent, i.e., compounds containing two or three carboxylate-containing anions per cation M.

As between otherwise identical compounds of formula I, those where Z is in other than lactone form are generally preferred over those wherein Z is a group of formula b.

Insofar as the compounds of Groups IAa, IBa, ICa, and IDa and each of the sub-groups thereof are concerned, the erythro isomers are preferred over the threo isomers, erythro and threo referring to the relative positions of the hydroxy groups in the 3- and 5-positions of the group of formula II.

Insofar as the compounds of Groups IAb, IBb, ICb, and IDb and each of the sub-groups thereof are concerned, the trans lactones are generally preferred over the cis lactones, cis and trans referring to the relative positions of $R_9$ and the hydrogen atom in the 6-position of the group of formula b.

The preferred stereoisomers of the compounds of formula I having only two centres of asymmetry wherein X is a direct bond, -CH=CH- or -$CH_2$CH=CH-, and Z is a group of formula a, are the 3R,5S isomer and the racemate of which it is a constituent, i.e., the 3R,5S-3S,5R (erythro) racemate.

The preferred stereoisomers of the compounds of formula I having only two centers of asymmetry wherein X is -$CH_2$-, $CH_2CH_2$-, -$CH_2CH_2CH_2$- or -CH=CH-$CH_2$-, and Z is a group of formula a, are the 3R,5R isomer and the racemate of which it is a constituent, i.e., the 3R,5R-3S,5S (erythro) racemate.

The preferences set forth in the preceding two paragraphs also apply to the compounds of formula I having more than two centers of asymmetry and represent the preferred configurations of the indicated positions.

The preferred stereoisomers of the compounds of formula I wherein X is a direct bond, -CH=CH- or -$CH_2$-CH=CH-, and Z is a group of formula b are the 4R,6S and 4R,6R isomers and the racemate of which each is a constituent, i.e., the 4R,6S-4S,6R (trans lactone) and 4R,6R-4S,6S (cis lactone) racemates with the 4R,6S isomer and the racemate of which it is a constituent being more preferred.

The preferred stereoisomers of the compounds of formula I wherein X is -$CH_2$-, -$CH_2CH_2$-, -$CH_2CH_2CH_2$- or -CH=CH-$CH_2$, and Z is a group of formula b are the 4R,6R and 4R,6S isomers and the racemate of which each is a constituent, i.e., the 4R,6R-4S,6S (trans lactone) and 4R,6S-4S,6R (cis lactone) racemates, with the 4R,6R isomer and the racemate of which it is a constituent being more preferred and the 4R,6R isomer being most preferred.

Each of the preferences set forth above applies unless otherwise stated, not only to the compounds of formula I, but also to the compounds of groups IA, IB, IC and ID and those of Groups IAa, IAb, IBa, IBb, ICa, ICb, IDa and IDb as well as to every other sub-group thereof set forth in the specification, e.g., Groups (i) et.seq., unless otherwise indicated. When any preference or group contains a variable, the preferred significances of that variable apply to the preference in question, unless otherwise indicated. Preferred groups of compounds include compounds:

(i) of Group IBa wherein $R_1$ is $R_{1Bx}$, $R_2$ is $R_{2Bx}$, $R_3$ is $R_{3Bx}$, $R_4$, $R_{4Bx}$, $R_9$ is $R'_9$, $R_{10}$ is $R'_{10}$, and X is X',

(ii) of (i) wherein $R_1$ is $R'_{1Bx}$, $R_2$ is $R'_{2BX}$, $R_3$ is $R'_{3Bx}$, $R_4$ is $R'_{4Bx}$, $R_9$ is hydrogen, $R_{10}$ is $R"_{10}$, and X is (E)-CH = CH-,

(iii) of (ii) wherein $R_1$ is $R"_{1Bx}$, $R_3$ is $R"_{3Bx}$, $R_4$ is $R"_{4Bx}$, and $R_1$ is $R"'_{10}$, preferably M,

(iv) of Group IBa wherein $R_1$ is $R_{1By}$, $R_2$ is $R_{2By}$, $R_3$ is $B_{3By}$, $R_4$ is $R_{4By}$, $R_9$ is $R'_9$, $R_{10}$ is $R'_{10}$, and X is X',

(v) of (iv) wherein $R_1$ is $R'_{1By}$, $R_2$ is $R'_{2By}$, $R_3$ is $R'_{3By}$, $R_4$ is $R'_{4By}$, $R_9$ is hydrogen, $R_{10}$ is $R"_{10}$, and X is (E)-CH = CH-,

(vi) of (v) wherein $R_2$ is $R"_{2By}$, $R_3$ is $R"_{3By}$, $R_4$ is $R"_{4By}$, and $R_{10}$ is $R"'_{10}$, preferably M,

(vii) of Group IBa wherein $R_1$ is $R_{1By}$, $R_2$, $R_{2By}$, $R_3$ is $R_{3Bx}$, $R_4$ is $R_{4Bx}$, $R_9$ is $R'_9$, $R_{10}$ is $R'_{10}$, and X is X',

(viii) of (vii) wherein $R_1$ is $R'_{1By}$, $R_2$ is $R'_{2By}$, $R_3$ is $R'_{3Bx}$, $R_4$ is $R'_{4Bx}$, $R_9$ is hydrogen, $R_{10}$ is $R"_{10}$, and X is (E)-CH = CH-,

(ix) of (viii) wherein $R_2$ is $R'_{2By}$, $R_3$ is $R"_{3Bx}$, $R_4$ is $R'_{4Bx}$, and $R_{10}$ is $R"'_{10}$, preferably M,

(x) - (xviii) of (i) - (ix) wherein the hydroxy groups in 3- and 5-positions of the group of formula a have the erythro configuration,

(xix) of Group IBb wherein $R_1$ is $R_{1Bx}$, $R_2$ is $R_{2Bx}$, $R_3$ is $R_{3Bx}$, $R_4$, $R_{4Bx}$, $R_9$ is $R'_9$, $R_{10}$ is $R'_{10}$, and X is X',

(xx) of (xix) wherein $R_1$ is $R'_{1Bx}$, $R_2$ is $R'_{2BX}$, $R_3$ is $R'_{3Bx}$, $R_4$ is $R'_{4Bx}$, $R_9$ is hydrogen, and X is (E)-CH = CH-,

(xxi) of (xx) wherein $R_1$ is $R"_{1Bx}$, $R_3$ is $R"_{3Bx}$, and $R_4$ is $R"_{4Bx}$,

(xxii) of Group IBb wherein $R_1$ is $R_{1By}$, $R_2$ is $R_{2By}$, $R_3$ is $R_{3By}$, $R_4$ is $R_{4By}$, $R_9$ is $R'_9$, and X is X',

(xxiii) of (xxii) wherein $R_1$ is $R'_{1By}$, $R_2$ is $R'_{2By}$, $R_3$ is $R'_{3By}$, $R_4$ is $R'_{4By}$, $R_9$ is hydrogen, and X is (E)-CH = CH-,

(xxiv) of (xxiii) wherein $R_2$ is $R"_{2By}$, $R_3$ is $R'_{3By}$, and $R_4$ is $R"_{4By}$,

(xxv) of Group IBb wherein $R_1$ is $R_{1By}$, $R_2$ is $R_{2By}$, $R_3$ is $R_{3Bx}$, $R_4$, $R_{4Bx}$, $R_9$ is $R'_9$, $R_{10}$ is $R'_{10}$, and X is X',

(xxvi) of (xxv) wherein $R_1$ is $R'_{1By}$, $R_2$ is $R'_{2By}$, $R_3$ is $R'_{3Bx}$, $R_4$ is $R'_{4Bx}$, $R_9$ is hydrogen, and X is (E)-CH = CH-,

(xxvii) of xxvi) wherein $R_2$ is $R"_{2By}$, $R_3$ is $R"_{3Bx}$ and $R_4$ is $R"_{4Bx}$,

(xxviii) - (xxxvi) of (xix) - (xxvii) wherein $R_9$ and the hydrogen atom in the 6-position of the group of Formula b are trans to each other, i.e., the trans lactones,

(xxxvii) of Group ICa wherein $R_1$ is $R_{1Cx}$, $R_2$ is $R_{2Cx}$, $R_3$ is $R_{3Cx}$, $R_4$ is $R_{4Cx}$, $R_9$ is $R'_9$ $R_{10}$ is $R'_{10}$, and X is X',

(xxxviii) of (xxxvii) wherein $R_1$ is $R'_{1Cx}$, $R_2$ is $R'_{2Cx}$, $R_3$ is $R'_{3Cx}$, $R_4$ is $R'_{4Cx}$, $R_9$ is hydrogen, $R_{10}$ is $R"_{10}$, and X is (E)-CH = CH-,

(xxxix) of (xxxviii) wherein $R_2$ is $R"_{2Cx}$, $R_3$ is $R"_{3Cx}$, $R_4$ is methyl and $R_{10}$ is $R"'_{10}$, preferably M,

(xl) of Group ICa wherein $R_1$ is $R_{1Cy}$, $R_2$ is $R_{2Cy}$, $R_3$ is $R_{3Cy}$, $R_4$ is $R_{4Cy}$, $R_9$ is $R'_9$, $R_{10}$ is $R'_{10}$, and X is X',

(xli) of (xl) wherein $R_1$ is $R'_{1Cy}$, $R_2$ is $R'_{2Cy}$, $R_3$ is $R'_{3Cy}$, $R_4$ is $R'_{4Cy}$, $R_9$ is hydrogen, $R_{10}$ is $R"_{10}$, and X is (E)-CH = CH-,

(xlii) of xli) wherein $R_1$ is $R"_{1Cy}$, $R_3$ is $R"_{3Cy}$, $R_4$ is $R"_{4Cy}$, and $R_0$ is $R"'_0$, preferably M,

(xliii) - (xlviii) of (xxxvii) - (xlii) wherein the hydroxy groups in the 3- and 5-positions of the group of Formula a have the erythro configuration,

(xlix) of Group ICb wherein $R_1$ is $R_{1Cx}$, $R_2$ is $R_{2Cx}$, $R_3$ is $R_{3Cx}$, $R_4$ is $R_{4Cx}$, $R_9$ is $R'_9$, and X is X',

(l) of (xlix) wherein $R_1$ is $R'_{1Cx}$, $R_2$ is $R'_{2Cx}$, $R_3$ is $R'_{3Cx}$, $R_4$ is $R'_{4Cx}$, $R_9$ is hydrogen, and X is (E)-CH = CH-,

(li) of (l) wherein $R_2$ is $R"_{2Cx}$, $R_3$ is $R"_{3Cx}$, and $R_4$ is methyl,

(lii) of Group ICb wherein $R_1$ is $R_{1Cy}$, $R_2$ is $R_{2Cy}$, $R_3$ is $R_{3Cy}$, $R_4$ is $R_{4Cy}$, $R_9$ is $R'_9$ and X is X',

(liii) of (lii) wherein $R_1$ is $R"_{1Cy}$, $R_2$. is $R'_{2Cy}$, $R_3$ is $R'_{3Cy}$, $R_4$ is $R'_{4Cy}$, $R_9$ is hydrogen. and X is (E)-CH = CH-,

(liv) of (liii) wherein $R_1$ is $R'_{1Cy}$, $R_3$ is $R"_{3Cy}$, and $R_4$ is $R"_{4Cy}$, and

(lv) - (lx) of (xlix) - (liv) wherein $R_{17}$ and the hydrogen atom in the 6-position of the group of Formula b are trans to each other, i.e. the trans lactones.

Groups (x) - (xviii) and (xliii) - (xlviii) embrace the 3R,5S-3S,5R racemate and the 3R,5S, and 3S,5R enantiomers of the compounds wherein X is -CH = CH-, the 3S,5R enantiomer being least preferred, and the 3R,5R-3S,5S racemate and the 3R,5R and 3S,5S enantiomers of the compounds wherein X is $-CH_2CH_2-$, the 3S,5S enantiomer being least preferred.

Groups (xxviii) - (xxxvi) and (lv) - (lx) embrace the 4R,6S-4S,6R racemate and the 4R,6S and 4S,6R enantiomers of the compounds wherein X is -CH = CH-, the 4S,6R enantiomer being least preferred, and the 4R,6R-4S,6S racemate and the 4R,6R and 4S,6S enantiomers of the compounds wherein X is $-CH_2CH_2-$, the 4S,6S enantiomer being least preferred.

Preferred groups of Formulae IA and ID include those that correspond to groups (xxxvii) - (lx) and the groups corresponing to groups (xxxvii)-(xxxix), (xliii)-(xlv), (xlix)-(li) and (lv)-(lvii) wherein any $R_{3cx}$ is $R_{3cy}$ (including 2-methyl-prop-1-en-1-yl), any $R\ \ _{cx}$ is $R'_{3cy}$ (including 2-methyl-prop-1-en-1-yl), and any $R"_{3cx}$ is $R"_{3cy}$ (including 2-methyl-prop-1-en-1-yl) i.e. groups (lxi)-(xcvi) (for group IA compounds) and (xcvii)-(cxxxii) (for group ID compounds). It goes without saying that none of these groups contain an $R_4$ substitutent.

Two groups of compounds of formula I are those wherein Ra is $R_1$, Rb is -X-Z, Rc is $R_2$, Rd is $R_3$ and Y is O or S, $R_1$, $R_2$ and $R_3$ are independently $C_{1-6}$alkyl not containing an asymmetric carbon atom, $C_{3-7}$cycloalkyl or a Ring B

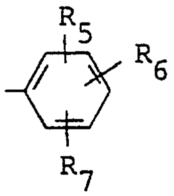

or in the case of $R_3$ additionally hydrogen, each $R_5$ is independently hydrogen, $C_{1-3}$alkyl, n-butyl, i-butyl, t-butyl, $C_{1-3}$alkoxy, n-butoxy, i-butoxy, trifluoromethyl, fluoro, chloro, phenyl, phenoxy or benzyloxy, each $R_6$ is independently hydrogen, $C_{1-3}$alkyl, $C_{1-3}$alkoxy, trifluoromethyl, fluoro, chloro, phenoxy or benzyloxy, and each $R_7$ is independently hydrogen, $C_{1-2}$alkyl, $C_{1-2}$alkoxy, fluoro or chloro with the proviso that there may only be one each of trifluoromethyl, phenoxy, or benzyloxy in each Ring B present, X is (E) CH=CH or $-CH_2-CH_2-$ and

Z is 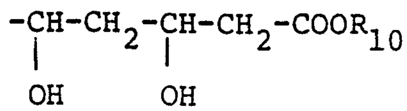 or

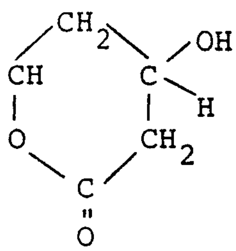

whereby $R_{10}$ is hydrogen, a physiologically acceptable ester group or a pharmaceutically acceptable cation.

Two other particular groups of compounds of formula I are those wherein Ra is -X-Z, Rb is $R_2$, Rc is $R_3$, Rd is $R_4$ and Y is 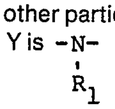 and those wherein Ra is $R_1$, Rb is -X-Z, Rc is $R_2$, Rd is $R_3$ and Y is 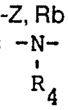 whereby $R_1$, $R_2$, $R_3$ and $R_4$ independently are $C_{1-6}$alkyl not containing an asymmetric carbon atom, $C_{3-7}$cycloalkyl or a Ring C

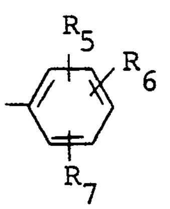

or in the case of $R_3$ and $R_4$ additionally hydrogen, each $R_5$ is independently hydrogen, $C_{1-3}$alkyl, n-butyl, i-butyl, t-butyl, $C_{1-3}$alkoxy, n-butoxy, i-butoxy, trifluoromethyl, fluoro, chloro, bromo, phenyl, phenoxy or benzyloxy, each $R_6$ is independently hydrogen, $C_{1-3}$alkyl, $C_{1-3}$alkoxy, trifluoromethyl, fluoro, chloro, phenoxy or benzyloxy, and each $R_7$ is independently hydrogen, $C_{1-2}$alkyl, $C_{1-2}$alkoxy, fluoro or chloro,
with the proviso that there may only be one each of trifluoromethyl, phenoxy or benzyloxy in each Ring C present, X is $(CH_2)_m$, -CH=CH-, $CH=CH-CH_2$ or $CH_2CH=CH$ and Z is

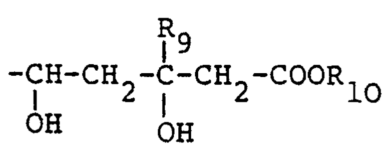

or 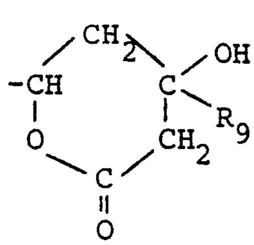

wherein $R_9$ is hydrogen or $C_{1-3}$alkyl and $R_{10}$ is hydrogen, a physiologically acceptable ester group or a pharmaceutically acceptable cation.

The compounds of formula I can be prepared by the following reactions where Q stands for

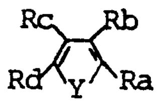

wherein Ra, Rb, Rc, Rd and Y are as defined above including provisos

a) when $R_9$ is hydrogen reducing a compound of formula VIII

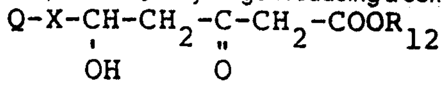

VIII

wherein $R_{12}$ is a radical forming an ester substantially inert to the reaction conditions and X is as defined above,

b) when $R_9$ is $C_{1-3}$alkyl hydrolysing a compound of formula XVI

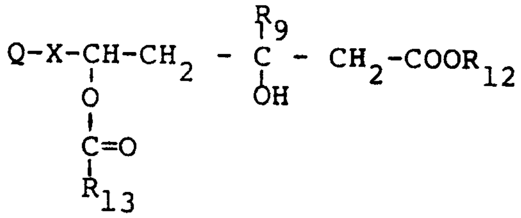

XVI

wherein $R_9$ is $C_{1-3}$alkyl, $R_{13}$ is part of an ester forming group and X and $R_{12}$ are as defined above,

c) when X is $-(CH_2)_2-$, $-(CH_2)_3-$, $-CH=CH-$ or $-CH_2CH=CH-$ deprotecting a compound of formula XXVII

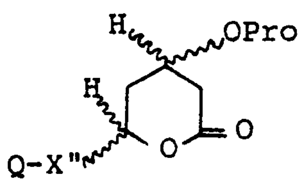

XXVII

wherein X" represents $-(CH_2)_2-$, $-(CH_2)_3-$, $-CH=CH-$ or $-CH_2CH=CH-$ and Pro is a protecting group,

d) when X is $-(CH_2)_2-$, $-(CH_2)_3-$, or $-(CH_2)_qCH=CH(CH_2)_q-$ deprotecting a compound of formula XI

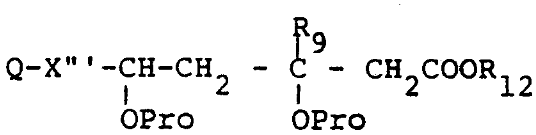

XXII

wherein X"' is $-(CH_2)_2-$, $-(CH_2)_3-$ or $-(CH_2)_q-CH=CH-(CH_2)_q-$, and q, $R_9$, $R_{12}$ and Pro are as defined above,

e) hydrolysing a compound of formula I in the form of an ester or a lactone,

f) esterifying or lactonising a compound of formula I in free acid form, or

g) esterifying a compound of formula I in lactone form, and when a free carboxyl group is present, recovering the compound obtained in free acid form or in the form of a salt. In processes a),b) and d) $R_{12}$ is preferably $C_{1-3}$alkyl, n-butyl, i-butyl, t-butyl or benzyl, more preferably $C_{1-3}$alkyl and especially $C_{1-2}$alkyl and $R_{13}$ is preferably $C_{1-3}$alkyl more preferably n-$C_{1-3}$alkyl, especially $C_{1-2}$alkyl.

Reduction according to a) is preferably carried out using a mild reducing agent such as sodium borohydride or, a complex of t-butylamine and borane in an inert organic solvent such as a lower alkanol, preferably ethanol, conveniently at a temperature of -10° to 30°C, under an inert atmosphere.

Use of an optically pure starting material will lead to only two optical isomers (diastereoisomers) of the resulting end product. However, if stereospecificity is desired it is preferred to utilize a stereo-selective reduction in order to maximize production of a mixture of the erythro stereoisomers (racemate) of which the preferred stereoisomer (as set forth above) is a constituent. Stereoselective reduction is preferably carried out in three steps. For example in the first step, the ketoester of formula VIII is treated with a tri(primary or secondary $C_{2-4}$alkyl)borane, preferably triethylborane or tri-n-butylborane, and optionally air to form a complex. The reaction temperature is suitably 0° to 50°C, preferably 0° to 25°C. The first step is carried out in an anhydrous inert organic solvent, preferably an ether solvent such as tetrahydrofuran or a mixture of tetrahydrofuran with methanol (preferably 3-4:1). In the second step, the complex is reduced with sodium borohydride, preferably in the same solvent as utilized for the first step, at -100° to -10°C, preferably -90° to

-70°C and then quenched e.g. with 10% HCl. In the third step, the product of the second step is, for example, treated with, preferably, anhydrous methanol at 20° to 60°C. The amount of methanol is not critical. However, a large excess, e.g., 50-100 moles per mole of ketoester of formula VIII, is typically utilized.

Hydrolysis according to b) or e) is carried out in a manner conventional for such reactions e.g. employing an inorganic hydroxide such as NaOH or KOH with, if desired subsequent acidification to give the free acid form. Suitable solvents are mixtures of water and water miscible solvents such as lower alkanols e.g. methanol or ethanol and reaction conveniently takes place at temperatures from 0°C to 75°C e.g. 20 to 70°C. If it is desired to recover the compound in a salt form corresponding to the cation of the hydroxide employed then slightly less than equivalent amounts of the latter may be employed. In b) $R_{13}$ will conveniently be the same as $R_{12}$ e.g. $C_{1-3}$alkyl more preferably n-$C_{1-3}$alkyl, especially $C_{1-2}$.

In process b) when Q is a pyrrole ring bearing a hydrogen atom on the nitrogen atom this will also bear a protecting group for example a further $-\underset{\underset{O}{\|}}{C}-R_{13}$ group. Preferably however in this case a protecting group of formula -Si($R_{34}$) ($R_{35}$) ($R_{36}$) is used instead of $-\underset{\underset{O}{\|}}{C}-R_{13}$ for both NH in the ring and OH in the side chain ($R_{34}$,$R_{35}$ independently = $C_{1-6}$alkyl especially methyl, $R_{36}$ = $C_{1-6}$alkyl especially methyl or t-butyl the latter being preferred). Such protection groups may be introduced and removed in conventional manner.

Lactonisation according to f) is carried out in conventional manner e.g. by heating the corresponding acid in an anhydrous inert organic solvent e.g. a hydrocarbon such as benzene, toluene or a xylene or mixtures thereof, preferably at temperatures of 75°C to reflux although more preferably not above 150°C. Preferably, however, a lactonisation agent, e.g. a carbodiimide, preferably a water-soluble carbodiimide such N-cyclohexyl-N'-[2'-(N"-methylmorpholinium)ethyl]carbodiimide p-toluenesulphonate, in an anhydrous inert organic solvent, e.g. a halogenated lower alkane, preferably methylene chloride is employed. Reaction temperatures then lie typically between 10° and 35°C, especially 20° to 25°C.

As is evident to those in the art, a racemic threo 3,5-dihydroxy-carboxylic acid yields a racemic cis lactone (two stereoisomers) and a racemic erythro 3,5-dihydroxycarboxylic acid yields a racemic trans lactone (two stereoisomers). Likewise if a single enantiomer of the 3,5-dihydroxycarboxylic acid is utilized, a single enantiomer of the lactone is obtained. For example, lactonisation of a 3R,5S erythro dihydroxycarboxylic acid yields a 4R,6S lactone.

Esterification according to f) is conventional, employing e.g. a large excess of a compound $R_{11}OH$, wherein $R_{11}$ is as defined above at 0-70°C, e.g. 20°C to 40°C in the presence of a catalytic amount of an acid such as p-toluenesulfonic acid. Where methyl esters are required these can also be obtained, e.g. using diazomethane in an anhydrous inert ether solvent such as tetrahydrofuran, 1,2-dimethoxyethane or 1,2-di-ethoxyethane and especially diethylether at e.g. 0° to 30°C, preferably 20° to 30°C.

Preferably, however, esterification takes place by first forming the corresponding lactone and reacting this with $M_2+$ $-OR_{11}$ ($M_2+$ = Na+ or K+) at 0° to 70°C preferably 20° to 25°C in an inert organic solvent e.g. an ether such as tetrahydrofuran or an alcohol of formula $R_{11}OH$ of a liquid, i.e.process g).

Process a) is preferably suited for compounds wherein X is $-(CH_2)_m-$ or (E)-CH=CH and in ester form.

Process b) is particularly suited for compounds wherein X is $(CH_2)_m-$ or (E)-CH=CH in salt form.

Process c) is particularly suited for compounds wherein X is -CH=CH- or $CH_2CH$=CH- and the lactone is in 4R,6S configuration and those wherein X is $-CH_2-CH_2-$ or $-CH_2CH_2CH_2-$ and the lactone is in 4R,6R configuration.

Process d) is particularly suited for compounds in ester form.

In processes c) and d) X" and X"' are unsaturated when $R_3$ is

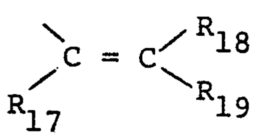 .

It will readily be appreciated that the various forms of the compounds of formula I may be interconverted as indicated in e), f) and g) above.

In the same way compounds obtained according to a), b), c) and d) may be hydrolysed to free acid forms and free acid forms may be esterified or lactonised to produce a desired end-product. The invention thus also provides a process for preparing a compound of formula I which comprises hydrolysing a compound of formula I in ester or lactone form or esterifying or lacto nising a compound of formula I in free acid form or esterifying a compound of formula I in lactone form and when a free carboxyl group is present recovering the compound obtained in free acid form or in the form of a salt.

Unless otherwise stated reactions are performed in a manner conventional for the type of reaction involved. Molar ratios and reaction times are as a rule conventional and non-critical and are chosen according to principles well established in the art on the basis of reactions and conditions employed.

Solvents, alone or as mixtures, are generally chosen which remain inert and liquid during the reaction in

question.

Examples of inert atmosphere are carbon dioxide and more usually nitrogen or a nobel gas, nitrogen being preferred. Most reactions, including those wherein use of an inert atmosphere is not mentioned, are carried out in such for convenience.

EP 114027 and 117228 and WO 86/00307 and 86/03488 including the examples thereof disclose analogous processes and further suitable reaction conditions.

Examples of protecting groups in reaction c) and d) are diphenyl-t-butylsilyl, tri-isopropylsilyl or dimethyl-t-butylsilyl, benzyl, tri-phenylmethyl, tetrahydrofuran-2-yl, tetra hydropyran-2-yl, 4-methoxytetra-hydrofuran-4-yl, n-($C_{2-6}$)alkanoyl. Especially preferred are trisubstituted silyl radicals in particular diphenyl-t-butylsilyl (= Pro').

Deprotection is carried out in conventional manner e.g. by cleavage under mild conditions such as employing e.g. for removal of a diphenyl-t-butylsilyl a fluoride reagent e.g. tetra-n-butyl-ammonium fluoride in an anhydrous inert organic medium preferably tetrahydrofuran containing glacial acetic acid at temperatures of 20° to 60° C, especially 20° to 25° C. Preferably 2 - 8 (for d) or 1 - 4 (for c) moles of fluoride are used with 1.2 to 1.5 moles of glacial acetic acid to each mole of fluoride.

The required starting materials may be prepared for example as illustrated in the following reaction schemes or in the examples hereinafter.

Further suitable reaction conditions are disclosed e.g. in EP 114027, 117228 and WO 86/00307 and 86/03488 including the examples thereof.

Abreviations:

AIO - anhydrous inert organic solvent
ES - ether solvent e.g. diethylether, 1,2-diethoxyethane, 1,2-dimethoxyethane, THF or mixtures thereof
HC - hydrocarbon solvent e.g. benzene, toluene, xylene or mixtures thereof
HLA - halogenated lower alkane solvent e.g. $CCl_4$, $CHCl_3$, 1,1-di-chloroethane, 1,2-dichloroethane, methylene chloride, 1,1,2-trichlorethane
THF - tetrahydrofuran
DMSO - dimethylsulfoxide
LDA - lithiumdiisopropylamide
nBuLi - n-butyllithium
DIBAH - diisobutylaluminium hydride
LAH - lithiumaluminium hydride
NBS - N-bromosuccinimide
DDQ - 2,3-dichloro-5,6-dicyano-1,4-benzoquinone

Lac is 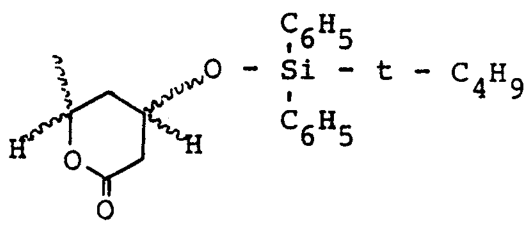

Pro' is 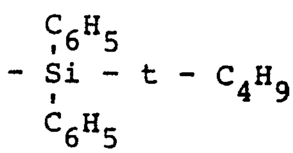

MTBE = methyl-t -butylether
TTPRD = tris-triphenylphosphineruthenium (II)chloride
RT = room temperature

In the reaction schemes:

$R_3'$ is Ring A ( with the proviso that none of $R_5$, $R_6$ and $R_7$ is fluoro, chloro, bromo or trifluoromethyl) preferably phenyl or p-tolyl or p-methoxyphenyl
$R_{4a}$ is as $R_4$ except for hydrogen
$R_{16}$ is n-($C_{1-4}$)alkyl especially ethyl
each $R_{20}$ is independently $C_{1-3}$alkyl preferably n-$C_{1-3}$alkyl especially $C_{1-2}$alkyl
each $R_{21}$ is independently $C_{1-2}$alkyl preferably the same $C_{1-2}$alkyl
Et is ethyl
$X_1$ is $-(CH_2)_m-$ or (E)-CH=CH-, especially (E)-CH=CH-, wherein m is 0, 1, 2 or 3,
$X_2$ is $-CH_2-$ or $-CH_2CH_2-$,

$X_3$ is a direct bond or $-CH_2-$,
$X_4$ is $-CH=CH-$, $-CH=CH-CH_2-$ or $-CH_2-CH=CH-$, preferably (E)-$CH=CH-$, (E)-$CH=CH-CH_2-$ or (E)-$CH_2-CH=CH-$ and especially (E)-$CH=CH-$,
$X_5$ is $-CH_2CH_2-$ or $-CH_2CH_2CH_2-$, especially $-CH_2CH_2-$.
$X_6$ is $-CH=CH-$ or $-CH_2-CH=CH-$, preferably $-CH=CH-$ and especially (E)-$CH=CH-$.
Y is chloro, bromo or iodo,
$Y^-$ is chloride, bromide or iodide,
Y' is chloro or bromo,
$Y'^-$ is chloride or bromide,
$M_2^+$ is sodium or potassium, and each of the other variables is as defined above.

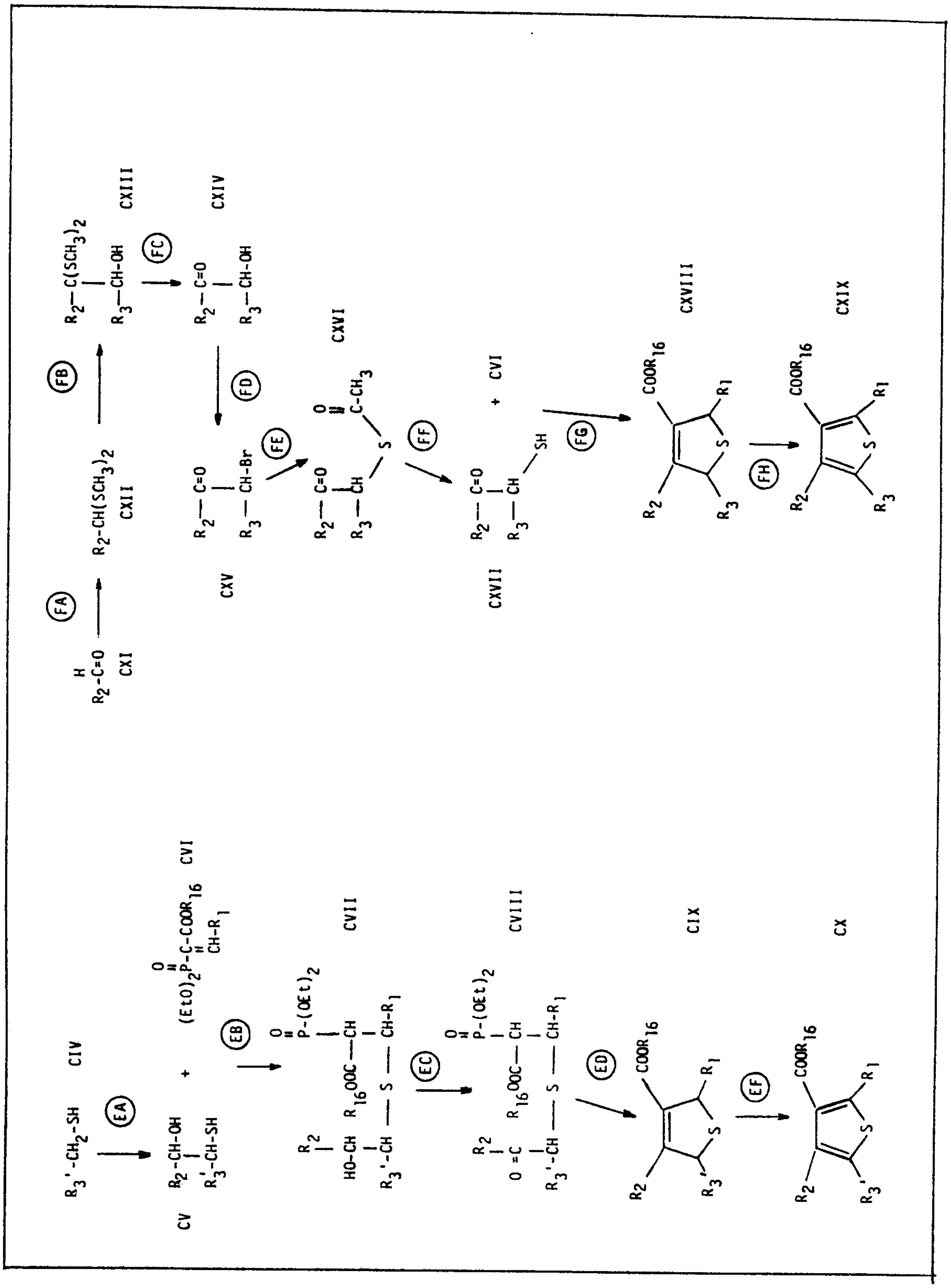


CIV CV CVI CVII CVIII CIX CX

CXI CXII CXIII CXIV CXV CXVI CXVII CXVIII CXIX

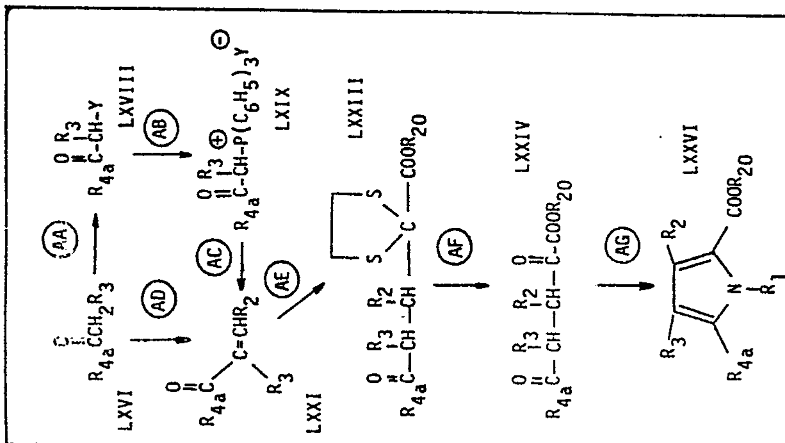

LXVI LXVIII LXIX LXXI LXXIII LXXIV LXXVI

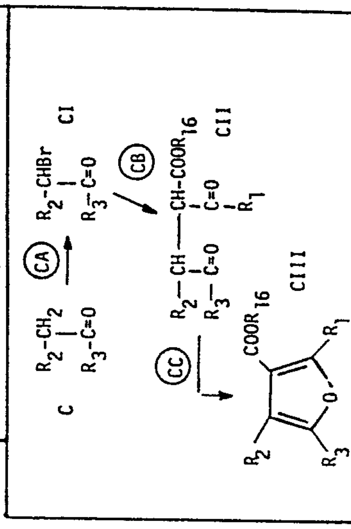

CI CII CIII

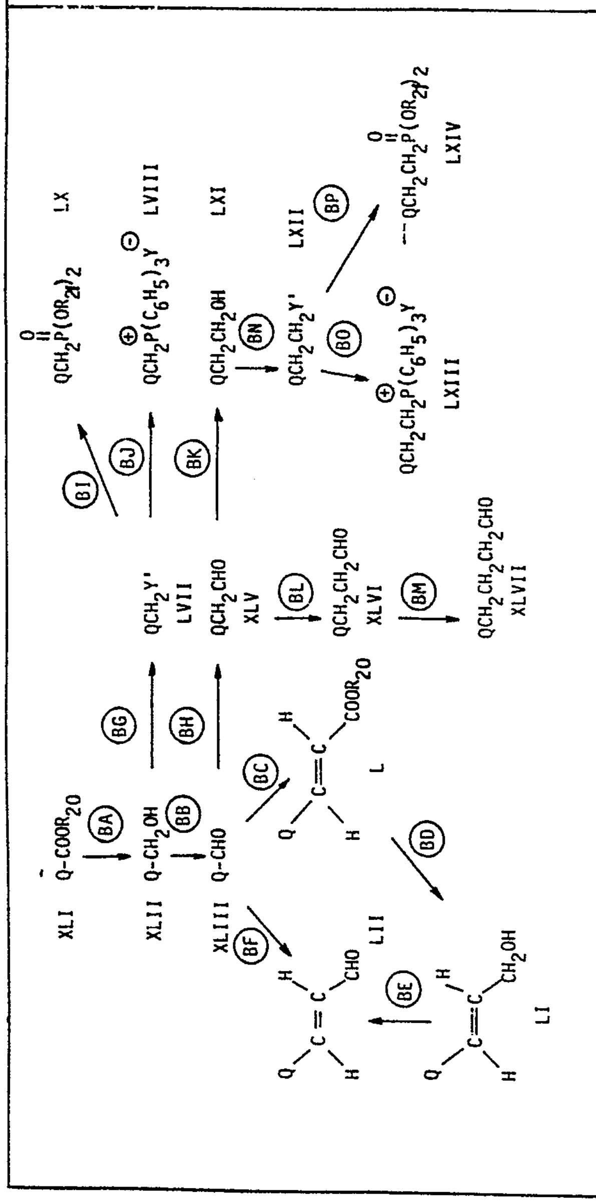

XLI XLII XLIII L LI LII LVII XLV XLVI XLVII LX LVIII LXI LXII LXIII LXIV

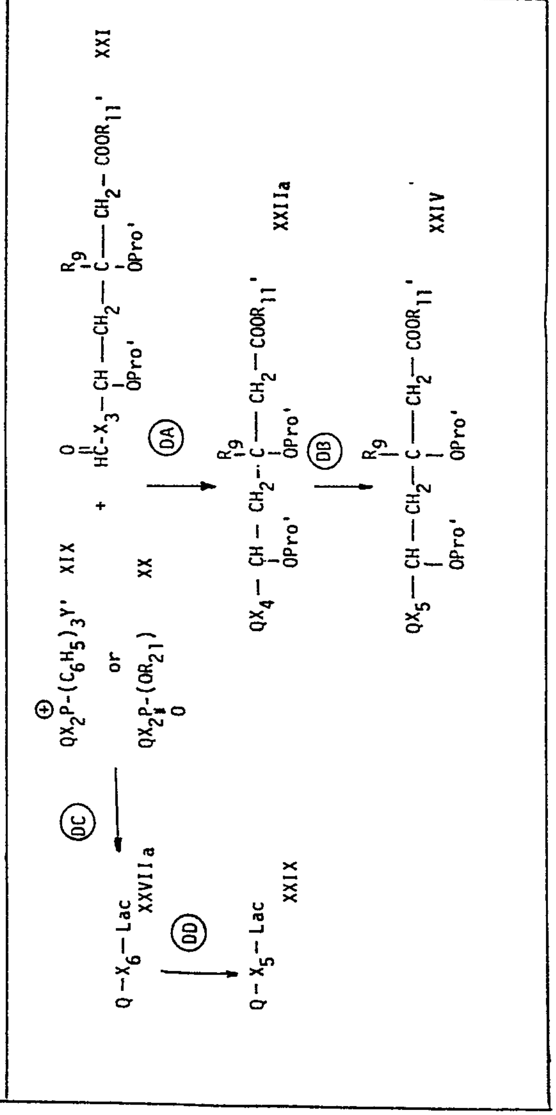

XIX XX XXI XXIIa XXIV XXVIIa XXIX

| REACTION | TYPE / STEPS | SPECIAL CONDITIONS/REACTANTS | TEMPERATURE | INERT ATMOS | SOLVENT |
|---|---|---|---|---|---|
| (AA) | Halogenation | $Y_2$ (preferably $Br_2$) | 0 to 40° | - | AIO preferably HLA e.g. $CHCl_3$ |
| (AB) | | $(C_6H_5)_3P$ | 20 to 40° pref. 20° to 25° | Yes | AIO preferably ES or HC especially toluene |
| (AC) | Wittig | 1.Base e.g. $NaOC_2H_5$ | 20° to 80° | Yes | AIO pref. lower alkanol esp. $C_2H_5OH$ |
| | | 2. Add $R_2CHO$ | 60° to 80° | Yes | —— " —— |
| (AD) | | 1.Base e.g. NaOH | 20° to 30° | - | lower alkanol preferably $C_2H_5OH$ + water |
| | | 2.Add $R_2CHO$ | 0° to 5° rising to 20° to 25° | - | —— " —— |
| (AE) | | 1) Strong base e.g. LDA + 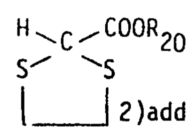 2)add LXXI and 3) quench with e.g. sat. $NH_4Cl$ | -80° to -60° pref. -78° | Yes (steps 1 + 2 only) | AIO pref. ES e.g. THF |
| (AF) | | $NBS/CH_3CN + H_2O$ 5-6 moles NBS per mole LXXIII | 0° | - | acetonitrile + water (pref. 4 : 1) |
| (AG) | | $R_1-NH_2 + TiCl_4$ | 0-120°, pref. 0° rising to reflux | Yes | AIO pref. HC esp. mixture of toluene and hexane |

| REACTION | TYPE / STEPS | SPECIAL CONDITIONS/REACTANTS | TEMPERATURE | INERT ATMOS | SOLVENT |
|---|---|---|---|---|---|
| (BA) | Reduction | LAH | -5 to 25° pref. -5 to 5° rising to 20 to 25° or 0 to 5° | Yes | AIO e.g. ES pref. THF or $(C_2H_5)_2O$ |
| (BB) | Oxidation | a) pyridinium-chlorochromate or -dichromate or chromium trioxide (pref. complexed with pyridine) or | 20 to 30° pref. 20 to 25° | Yes | a) AIO pref. HLA esp. $CH_2Cl_2$ |
| | | b) activated manganese dioxide or | — " — | Yes | b) AIO pref. HLA or ES esp. $(C_2H_5)_2O$ |
| | | c) N-methylmorpholine-N-oxide-$1H_2O$ + catalytic amount of $((C_6H_5)_3P)_3$ ruthenium (II) chloride each in large molar excess | — " — | Yes | c) anh. acetone |
| (BC) | Wittig | a) $(C_6H_5)_3P$=CH-$COOR_{20}$ (XLVIII) | 80° to reflux | Yes | AIO pref. HC esp. toluene |
| | | b) 1) $(R_{21}O)_2PO$-$CH_2COOR_{20}$ (XLIX) + strong base esp.NaH | -20 to 25° pref. -20 to 0° | Yes | AIO, pref. ES esp. THF |
| | | 2) add XLIII | -20 to 65° | Yes | — " — |
| (BD) | Reduction | e.g. LAH or DIBAH. quench with $H_2O$, sat. $NH_4Cl$ or aq. $Na_2SO_4$ solution | -78 to 25° pref -78 to 25° | Yes<br>- | AIO pref. ES esp. THF<br>- |

| REACTION | TYPE / STEPS | SPECIAL CONDITIONS/REACTANTS | TEMPERATURE | INERT ATMOS | SOLVENT |
|---|---|---|---|---|---|
| (BE) | Oxidation | as (BB) b) and pref. (BB) c) | as (BB) | Yes | as (BB) b) and c) |
| (BF) | Addition | 1) $\overset{\oplus}{L}i\ [CH=CH-OC_2H_5]^{\ominus}$ then quench | -80 to -40° pref. -60° to -80° | Yes | AIO pref. ES esp. THF |
| | | 2) catalytic amount of p.TSOH-$H_2O$ | 20-40° pref. 20 to 25° | - | Mixture of ES + $H_2O$, pref. THF + $H_2O$ |
| (BG) (BN) | Halogenation | SOY'$_2$ or PY'$_3$ | -10 to -80° | - | AIO pref. ES e.g. $(C_2H_5)_2O$ or THF; HLA e.g. $CH_2Cl_2$ or HC e.g. benzene |
| (BH) (BL) (BM) | Wittig | 1) $(C_6H_5)_3P^{\oplus}-CH_2OCH_3Cl^{\ominus}$ (XLIV) + strong base e.g. NaH, phenyllithium or pref. n.BuLi | -40 to 0° pref. -35 to -20° | Yes | AIO pref. ES e.g. THF |
| | | 2) Add XLIII | -30 to 0° pref. -20 to 0° | Yes | ——— " ——— |
| | | 3) Hydrolyse with strong acid e.g. 70% perchloric in large molar excess | 0 to 30° | - | aqueous acid + ES (e.g. THF) |
| (BI) (BP) | | $P(OR_{21})_3$ (LIX) | 20 to 140° usually 110-140° | Yes | HC e.g. benzene or xylene or excess LIX |

| REACTION | TYPE / STEPS | SPECIAL CONDITIONS/REACTANTS | TEMPERATURE | INERT ATMOS | SOLVENT |
|---|---|---|---|---|---|
| (BJ) (BO) | | excess $(C_6H_5)_3P$ | 60 to reflux pref. $\leq$150° | Yes | AIO pref. abs. $C_2H_5OH$ |
| (BK) | | as a) mild reducing agent | as a) | as a) | as a) |
| (CA) | Bromination | $Br_2$ | 0 to 40° | - | AIO e.g. HLA e.g. $CHCl_3$ |
| (CB) | Addition | $[R_1-\overset{O}{\overset{\|}{C}}-CH_2-\overset{O}{\overset{\|}{C}}-OR_{16}]^{\ominus} M^{\oplus}$ ($M^{\oplus}=Li^{\oplus}$, $Na^{\oplus}$ or $K^{\oplus}$, pref. $Na^{\oplus}$) pref. prepared in situ | 0 to 40° pref. 0° $\rightarrow$ RT | - | AIO e.g. ES e.g. THF |
| (CC) | Cyclisation | $BF_3(C_2H_5)_2O$ (pref. molar excess) quench with e.g. aq. $NaHCO_3$ | 80° to 140° | - | AIO e.g. HC e.g. toluene or xylene |
| (DA) | Wittig | A) 1) strong base e.g. NaH or pref. n.BuLi + XIX | -40 to 5° pref. -35 to -20° | Yes | AIO e.g. HC as toluene or pref. ES e.g. THF |
| | | 2) add XXI or | -55 to 25° pref -35 to -5° | Yes | ——— " ——— |
| | | B) 1) strong base pref. nBuLi or LDA + XX + optionally LiCl | -10 to 0° | Yes | AIO pref. ES e.g. THF |
| | | 2) add XXI | -10 to 0° | Yes | ——— " ——— |
| (DB) (DD) | Hydrogenation | excess $H_2$ + $PtO_2$ catalyst | 20 to 25° | - | lower alkanol e.g. $C_2H_5OH$ |

| REACTION | TYPE / STEPS | SPECIAL CONDITIONS/REACTANTS | TEMPERATURE | INERT ATMOS | SOLVENT |
|---|---|---|---|---|---|
| (DC) | Wittig using LacCHO in place of XXI | as (DA) | as (DA) | Yes | as (DA) |
| (EA) | | 1) alkali metal salt of CIV ($R_{16}$ = Et) using e.g. nBuLi | -10 to +10° | - | AIO pref. ES e.g. THF |
| | | 2) add $R_2$-CHO quench with e.g. sat. aq. $NH_4Cl$ | -30 to -15° | - | ——— " ——— |
| (EB) | | CV + CVI + acid neutralising agent e.g. tert.amine e.g. $(C_2H_5)_3N$ | 15 to 60° e.g. RT | - | AIO pref. ES e.g. THF |
| (EC) | Oxidation | 1) conventional e.g. Swern's reaction (oxalylchloride + DMSO) | -90 to -40° | - | AIO pref. HLA e.g. $CH_2Cl_2$ |
| | | 2) add $(C_2H_5)_3N$. quench e.g. with water | 20 to 70° | | ——— " ——— |
| (ED) | Cyclisation | strong base e.g. LDA quench e.g. with aq. $NH_4Cl$ | -10 to 5° | | AIO pref. ES e.g. THF |

| REACTION | TYPE / STEPS | SPECIAL CONDITIONS/REACTANTS | TEMPERATURE | INERT ATMOS | SOLVENT |
|---|---|---|---|---|---|
| (EF) (FH) | Aromatisation | DDQ | 20 to 40° | - | AIO pref. e.g. HLA e.g. $CH_3Cl$ or ES e.g. THF |
| (FA) | | 2 mol $CH_3SH$ + $BF_3$ $(C_2H_5)_2O$ quench with e.g. dil. aq. NaOH | -10 to 5° | | AIO e.g. ES |
| (FB) | | 1) CXII + strong base e.g. n-BuLi | -80 to -60° e.g. -78° | - | AIO pref. ES e.g. THF |
| | | 2) add $R_3C(H)=O$ quench with sat.aq.$NH_4Cl$ | —"— | - | —"— |
| (FC) | | red mercuric oxide + $BF_3(C_2H_5)_2O$ | -10 to 5° | - | |
| (FD) | Bromination | $CBr_4$ + $(C_6H_5)_3P$ | 20 to 60° | | AIO e.g. ES e.g. THF |
| (FE) | | $KSC(=O)-CH_3$ | 20 to 50° | | lower alkanol e.g. $C_2H_5OH$ |
| (FF) | | strong acid e.g. conc. HCl | 20 to 80° | | lower alkanol e.g. $CH_3OH$ |
| (FG) | | 1) CXVII + strong base e.g. LDA | e.g. 0° | | AIO pref. ES e.g. THF |
| | | 2) add CVI | -80 to -60° e.g. -78° | | —"— |
| | | quench with sat. aq. $NH_4Cl$ | -80° to 25° | | |

The conditions given hereinabove are largely conventional for such reactions and can be varied in conventional manner according to the particular intermediates/end products. This applies e.g. to molar ratios,

temperature, reaction times and the like which are chosen according to principles well established in the art on the basis of reactants and conditions employed.

Intermediates, the production of which is not described above are known or may be prepared by or analogously to known methods e.g. as described in EP 114027 and 117228 or WO 86/00307 (US Pat. 4,613,610) and 86/03488 including the examples thereof. This applies for example to intermediates VIII,XVI,XXII and XXVII.

Reaction products, both intermediates and final, can be isolated (e.g. from compound mixtures or reaction mixtures) and purified in conventional manner whereby intermediates can, where appropriate, be employed directly in a subsequent reaction.

Mixtures of stereoisomers (cis, trans and optical) can be separated by conventional means at whatever stage of synthesis is appropriate. Such methods include re-crystallisation, chromatography, formation of esters with optically pure acids and alcohols or of amides and salts with subsequent reconversion under retention of optical purity. For example diastereoisomeric (-)-α-naphthylphenylmethylsilyl derivatives of a lactone type end product of formula I may be separated by conventional means. Such separations are described in US Pat. 4,613,610.

Salts may be prepared in conventional manner from free under, lactones and esters and vice-versa. Whilst all salts are covered by the invention pharmaceutically acceptable salts especially sodium, potassium and ammonium particularly sodium salts are preferred.

The various forms of the compounds of formula I are by virtue of their interconvertability useful as intermediates in addition to the use set out below.

Also within the scope of this invention are the intermediates of formulae VIII, XVI, XIX, XX, XXII, LXXIIa, XXIV, XXVII, XXVIIa, XXIX, XLV-XLVII, L-LII, LVIII, LX, LXIII, LXIV.

The preferences for each variable are the same as set out for formula I and preferred groups of compounds correspond to those listed for formula I as appropriate to and consistent therewith.

The compounds of formula I possess pharmacological activity in particular as competitive inhibitors of 3-hydroxy-3-methyl-glutaryl coenzyme A (HMG-CoA) reductase and as a consequence are inhibitors of cholesterol biosynthesis as demonstrated in the following tests.

Test A: In Vitro Microsomal Assay of HMG-CoA Reductase Inhibition: As described in EP 114027 and USP 4,613,610

Concentration of test substance 0.0005 to 2,000 μmolar.

Test B: In Vivo Cholesterol Biosynethesis Inhibition: As described in EP 114027 and USP 4,613,610

Dosage of test substance 0.01 to 200 mg/kg body weight.

The compounds are thus indicated for use as hypolipoproteinemic and anti-atherosclerotic agents.

An indicated suitable daily dosage for use in the treatment of hyperlipoproteinemia and atherosclerosis is from about 0.5 to 2000 mg preferably 0.5 to 200 mg suitably administered in divided dosages two to four times daily or in retard form. A typical dosage unit for oral administration may contain 0.01 to 500 mg typically 0.25 to 500 mg.

The compounds of formula I may administered in similar manner as known compounds suggested for use in such indications e.g. Compactin or Mevinolin. The suitable daily dosage for a particular compound will depend on a number of factors such as its relative potency of activity. It has, for example been determined that the preferred compounds of Examples 2 and 9 and compound 19 obtained an $ED_{50}$ of 0.53 mg/kg, 0.17 mg/kg and 0.076 mg/kg respectively in Test B compared with 3.5 mg/kg for Compactin and 0.41 mg/kg for Mevinolin. It is therefore indicated that these compounds may be administered at similar or significantly lower dosages than conventionally proposed for Compactin or Mevinolin.

They may be administered in free acid form or in the form of a physiologically-acceptable ester e.g. one which is also physiologically hydrolysable or a lactone thereof or in pharmaceutically acceptable salt form.

The invention therefore also concerns a method of treating hyperlipoproteinemia or atherosclerosis by administration of a compound of formula I in free acid form or in the form of a physiologically-hydrolysable and -acceptable ester or a lactone thereof or in pharmaceutically acceptable salt form as well as such compounds for use as pharmaceuticals e.g. as hypolipoproteinemic and anti-atherosclerotic agents.

The compounds may be administered alone, or in admixture with a pharmaceutically acceptable diluent or carrier, and, optionally other excipients, and administered orally in such forms as tablets, elixirs, capsules or suspensions or parenterally in such forms as injectable solutions or suspensions.

The preferred pharmaceutical compositions from the standpoint of ease of preparation and administration are solid compositions, particularly tablets and hard-filled or liquid-filled capsules.

Such compositions also form part of the invention.

The following examples, in which all temperatures are in °C illustrate the invention.

Example 1

Ethyl (±)-erythro-(E)-3,5-dihydroxy-7-[1'-(4"-fluorophenyl)-3'-(1"-methylethyl)-5'-phenyl-1H-pyrrol-2'-yl]hept-6-enoate (Compound No. 1)

(Type IBa, X = (E)CH = CH, $R_9$ = H, $R_{10}$ = $C_2H_5$, $R_2$ = i-propyl, $R_3$ = hydrogen, $R_4$ = phenyl, $R_1$ = p-fluorophenyl)

Step 1 (Reaction AB)

(2-Oxo-2-phenylethyl)triphenylphosphonium bromide (Compound (CCII)

50.0 g (251 mmoles) of a-bromoacetophenone Compound (CCI) and 65.9 g (251 mmoles) of triphenylphosphine are stirred in 500 ml. of toluene at 20°-25° under nitrogen for 16 hours, and the resulting solid is collected by filtration, rinsed with toluene and rinsed with diethyl ether to obtain the product as a white powder.

Step 2 (Reaction AC)

(E)-4-Methyl-1-phenylpent-2-en-1-one (Compound CCIV)

A solution of 6.64 g. (289 mmoles) of sodium in 250 ml. of absolute ethanol is quickly added dropwise to a suspension of 133.3 g. (289 mmoles) of Compound CCII in 1 l. of absolute ethanol stirred at 20°-25° C., the suspension is gently warmed until a clear solution results, 31.5 ml. (25.0 g.; 347 mmoles) of isobutyraldehyde is added dropwise to the solution, and the reaction mixture is refluxed for 16 hours and cooled to 20°-25° C., the reaction mixture being maintained under dry nitrogen throughout. The reaction mixture is evaporated at reduced pressure, the resulting oily solid is triturated with diethyl ether, and the insoluble material is removed by filtration. The diethyl ether solution is washed with water, washed twice with saturated sodium chloride solution, dried over anhydrous magnesium sulfate, filtered and evaporated at reduced pressure. The residue is triturated with diethyl ether, any insoluble material is removed by filtration, and the diethyl ether is evaporated at reduced pressure to obtain a clear yellow liquid which is fractionally vacuum distilled through a vacuum jacketed Vigreux column to obtain the product as a clear liquid b.p. 90°-92°C./~ 1 mm. Hg.

Step 3 (Reaction AE)

Ethyl (±)-2-[1'-(1"-methylethyl)-2'-oxo-2'-phenylethyl]-1,3-dithiolane-2-carboxylate (Compound CCVI)

83 ml. of 1.65M. n-butyllithium/hexane (138 mmoles) is added via syringe to a solution of 20.2 ml. (14.58 g.; 144 mmoles) of diisopropylamine in 200 ml. of dry tetrahydrofuran (distilled from ketyl) stirred at -78° C., the reaction mixture is warmed to 0°C. and cooled to -78°C., 18.7 ml. (23.35 g.; 131 mmoles) of ethyl 1,3-dithiolane-2-carboxylate (Compound CCV) is added dropwise to the reaction mixture stirred at -78° C., the reaction mixture is stirred at -78° C. for 30 minutes, a solution of 22.749 g. (131 mmoles) of Compound CIV in 50 ml. of dry tetrahydrofuran (distilled from ketyl) is added dropwise with stirring at -78°C., and the reaction mixture is stirred at -78° C. for 3 hours, the reaction mixture being maintained under dry nitrogen throughout. The reaction mixture is quenched at -78°C. with saturated ammonium chloride solution and warmed to 20°-25° C., the tetrahydrofuran is evaporated at reduced pressure, and the residue is partitioned between ethyl acetate and water. The aqueous phase is acidified to about pH 2 with 10% hydrochloric acid and extracted with ethyl acetate. The ethyl acetate layers are combined, washed twice with saturated sodium chloride solution, dried over anhydrous magnesium sulfate, filtered and evaporated at reduced pressure. The residue is triturated with diethyl ether to obtain the crude product as a white solid.

Step 4 (Reaction AF)

Ethyl (±)-2,5-dioxo-3-(1'-methylethyl)-5-phenylpentanoate (Compound CCVII)

A solution of 27.1 g. (76.9 mmoles) of crude Compound CCVI from Step 3 in 200 ml. of acetonitrile is added dropwise to a solution of 82.1 g. (461.3 mmoles) of N-bromosuccinimide in 500 ml. of a 4:1 mixture of acetonitrile and water stirred at 0° C., the reaction mixture is stirred at 0° C. for 4.5 hours, sufficient saturated sodium carbonate solution is added to obtain a clear solution, the acetonitrile is evaporated at reduced pressure, the residue is partitioned between ethyl acetate and water, the aqueous phase is acidified to about pH 2 with 10% aqueous hydrochloric acid and extracted with ethyl acetate, and the ethyl acetate layers are combined, washed twice with saturated sodium chloride solution, dried over anhydrous magnesium sulfate, filtered and evaporated at reduced pressure to obtain the crude product as a clear yellow liquid.

Step 5 (Reaction AG)

Ethyl 1-(4'-fluorophenyl)-3-(1'-methylethyl)-5-phenyl-1H-pyrrole-2-carboxylate (Compound CCIX)

24.59 g. (89 mmoles) of crude Compound CCVII from Step 4 and 16.9 ml. (19.8 g.; 178 mmoles) of 4-fluoroaniline are stirred at 0° C. in 350 ml. of toluene, a solution of 5.9 ml. (10.13 g.; 53.4 mmoles) of titanium tetrachloride in 50 ml. of hexane is added dropwise with stirring at 0° C., and the reaction mixture is allowed to slowly warm to 20°-25° C. and refluxed for 16 hours, the reaction mixture being maintained under dry nitrogen throughout. The reaction mixture is filtered through a pad of Celite, the Celite is rinsed with ethyl acetate, and the rinse and filtrate are combined and evaporated at reduced pressure to obtain a brown solid.

The brown solid is recrystallized from absolute ethanol to obtain slightly colored needles, a second crop is obtained from the absolute ethanol, and the two crops are combined and recrystallized from absolute ethanol to obtain the product as a white powder.

A small sample is dissolved in the minimum amount of methylene chloride and flash chromatographed on

250 g. of 230-400 mesh A.S.T.M. silica gel utilizing 25% diethyl ether/hexane as the eluant, the eluant is evaporated, and the residue is recrystallized from absolute ethanol to obtain an analytical sample, m.p. 107°-110°C.

Step 6 (Reaction BA)

1-(4'-Fluorophenyl)-3-(1'-methylethyl)-5-phenyl-1H-pyrrole-2-methanol (Compound CCX)

A solution of 15.0 g. (42.7 mmoles) of Compound CCIX in 100 ml. of dry tetrahydrofuran (distilled from ketyl) is added dropwise to a suspension of 4.86 g. (128.1 mmoles) of lithium aluminum hydride in 150 ml. of dry tetrahydrofuran (distilled from ketyl) stirred at 0°C., and the reaction mixture is allowed to warm to 20°-25°C., stirred at 20°-25°C. for 3 hours and cooled to 0°C., the reaction mixture being stirred under dry nitrogen throughout. The reaction mixture is quenched at 0°C. with water, the white gum is removed by filtration, the small aqueous phase is separated and the organic phase is washed twice with saturated sodium chloride solution, dried over anhydrous magnesium sulfate, filtered and evaporated at reduced pressure to obtain a white solid which is recrystallized from diethyl ether/hexane to obtain the product as a white powder. The mother liquor is evaporated at reduced pressure, and the residue is recrystallized from methylene chloride/hexane to obtain a second crop.

Step 7 (Reaction BB)

1-(4'-Fluorophenyl)-3-(1'-methylethyl)-5-phenyl-1H-pyrrole-2-carboxaldehyde (Compound CCXII)

A solution of 7.8 g. (25.2 mmoles) of Compound CCX in 200 ml. of dry acetone (dried over 4Å. molecular sieves) is added dropwise to a mixture of 483 mg. (0.5 mmole) of tris-(triphenylphosphine)ruthenium(II) chloride (Compound CCXI) and 8.62 g. (50.4 mmoles) of N-methylmorpholine-N-oxide monohydrate in 100 ml. of dry acetone (dried over 4Å. molecular sieves) stirred at 20°-25°C., and the reaction mixture is stirred at 20°-25°C. for 4 hours, the reaction mixture being maintained under dry nitrogen throughout. The reaction mixture is filtered through 230-400 mesh A.S.T.M. silica gel, the silica gel is rinsed with diethyl ether, and the rinse and filtrate are combined and evaporated at reduced pressure to obtain a tan solid. The tan solid is dissolved in the minimum amount of methylene chloride and flash chromatographed on 450 g. of 230-400 mesh A.S.T.M. silica gel utilizing 25% diethyl ether/hexane as the eluant, and the eluant is evaporated at reduced pressure to obtain the product as a flocculant white solid m.p. 135°-139°C.

Step 8 (Reaction BC)

Ethyl (E)-3-[1'-(4"-fluorophenyl)-3'-(1"methylethyl)-5'-phenyl-1H-pyrrol-2'-yl]prop-2-enoate (Compound CCXIV)

1.03 g. of 60% sodium hydride/mineral oil (25.7 mmoles) is washed twice with hexane and suspended in 100 ml. of dry tetrahydrofuran (distilled from ketyl), the suspension is stirred at 20°-25°C., approximately 1 ml. of triethyl phosphonoacetate (Compound CCXIII) is added to initiate the reaction, the reaction mixture is cooled to -20°- -15°C., approximately 3.9 ml. of triethyl phosphonoacetate is added dropwise with stirring at -20°- -15°C. (the total amount of triethyl phosphonoacetate being 4.9 ml. (5.48 g.; 24.45 mmoles)), the reaction mixture is stirred at -20°- -15°C. for 1 hour, a solution of 5.0 g. (16.3 mmoles) of Compound CCXII in 50 ml. of dry tetrahydrofuran (distilled from ketyl) is added dropwise with stirring at -20°- -15°C., and the reaction mixture is allowed to warm to 20°-25°C., refluxed for 3 hours and stirred at 20°-25°C. for 16 hours, the reaction mixture being maintained under dry nitrogen throughout. The reaction mixture is diluted with diethyl ether and extracted with water, and the aqueous phase is reexctracted with diethyl ether. The organic phases are combined, washed twice with saturated sodium chloride solution, dried over anhydrous magnesium sulfate, filtered and evaporated at reduced pressure to obtain a white solid which is recrystallized from diethyl ether to obtain the product as a white powder. A second crop is obtained from diethyl ether/hexane.

An analytical sample is obtained by recrystallization of a small sample from diethyl ether/hexane, m.p. 145°-147.5°C.

Step 9 (Reaction BD)

(E)-3-(1'-(4"-Fluorophenyl)-3'-(1"-methylethyl)-5'-phenyl-1H-pyrrol-2'-yl]prop-2-en-1-ol (Compound CCXV)

44 ml. of 1.5M. diisobutylaluminum hydride/toluene (66 mmoles) is added dropwise to a solution of 5.0 g. (13.25 mmoles) of Compound CCXIV in 100 ml. of dry tetrahydrofuran (distilled from ketyl) stirred at -78°C., and the reaction mixture is stirred at -78°C. for 1 hour, the reaction mixture being stirred under dry nitrogen throughout. The reaction mixture is quenched at -78°C. with water and warmed to 20°-25°C., sufficient 10% hydrochloric acid is added to dissolve the gel, and the mixture is extracted twice with diethyl ether. The diethyl ether extracts are combined, washed with saturated sodium bicarbonate solution, washed twice with saturated sodium chloride solution, dried over anhydrous magnesium sulfate, filtered and evaporated at reduced pressure. The residue is recrystallized from diethyl ether to obtain the product as a white powder, m.p. 142°-148°C. The mother liquor is evaporated at reduced pressure, the residue is dissolved in the minimum amount of methylene chloride and flash chromatographed on 150 g. of 230-400 mesh A.S.T.M. silica

gel utilizing 50% diethyl ether/hexane as the eluant. and the eluant is evaporated at reduced pressure to obtain additional product as a tan powder.

Step 10 (Reaction BE)

(E)-3-[1'-(4"-Fluorophenyl)-3'-(1"-methylethyl)-5'-phenyl-1H-pyrrol-2'-yl]prop-2-en-1-al (Compound CCXVI)

A solution of 3.3 g. (9.84 mmoles) of Compound CCXV in 150 ml. of dry acetone (dried over 4Å. molecular sieves) is added dropwise to a mixture of 283 mg. (0.3 mmole) of tris-(triphenylphosphine)ruthenium(II) chloride (Compound CCXI) and 2.31 g. (19.68 mmoles) of N-methylmorpholine-N-oxide•monohydrate in 100 ml. of dry acetone (dried over 4Å. molecular sieves) stirred at 20°-25°C., the reaction mixture is stirred at 20°-25°C. for 3 hours, an additional 1.15 g. (9.84 mmoles) of N-methylmorpholine-N-oxide•monohydrate is added, the reaction mixture is stirred at 20°-25°C. for 2 hours, an additional 189 mg. (0.2 mmole) of tris(triphenylphosphine)-ruthenium(II) chloride is added, and the reaction mixture is stirred at 20°-25°C. for 16 hours, the reaction mixture being stirred under dry nitrogen throughout. The reaction mixture is filtered through a pad of Celite and evaporated at reduced pressure to obtain a dark green oily solid which is dissolved in the minimum amount of methylene chloride and flash chromatographed on 250 g. of 230-400 mesh A.S.T.M. silica gel utilizing 25% diethyl ether/hexane as the eluant. The eluant is evaporated at reduced pressure to obtain the product as a yellow solid m.p. 180°-184°C.

Step 11

Ethyl (±)-(E)-7-[1'-(4"-fluorophenyl)-3'-(1"-methylethyl)-5'-phenyl-1H-pyrrol-2'-yl]-5-hydroxy-3-oxohept-6-enoate (Compound CCXVIII)

528 mg. (13.2 mmoles) of 60% sodium hydride/mineral oil is washed twice with hexane and suspended in 50 ml. of dry tetrahydrofuran (distilled from ketyl), the suspension is cooled to -20°- -15°C., 1.53 ml. (1.56 g.; 12.0 mmoles) of ethyl acetoacetate is added dropwise, the reaction mixture is stirred at -20°- -15°C. for 30 minutes, 7.6 ml. of 1.65M. n-butyllithium/hexane (12.54 mmoles) is added dropwise with stirring at -20°- -15°C., the reaction mixture is stirred at -20°- -15°C. for 15 minutes, a solution of 2.0 g. (6.0 mmoles) of Compound CCXVI in 50 ml. of dry tetrahydrofuran (distilled from ketyl) is added dropwise with stirring at -15°C., and the reaction mixture is stirred at -15°C. for 2.5 hours, the reacttion mixture being stirred under dry argon throughout. The reaction mixture is quenched at -15°C. with saturated ammonium chloride solution and warmed to 20°-25°C., the tetrahydrofuran is evaporated at reduced pressure, the residue is partitioned between diethyl ether and water, the aqueous phase is extracted with diethyl ether, and the diethyl ether phases are combined, washed twice with saturated sodium chloride solution, dried over magnesium sulfate, filtered and evaporated at reduced pressure to a brown oil. The brown oil is dissolved in the minimum amount of methylene chloride and flash chromatographed on 250 g. of 230-400 mesh A.S.T.M. silica gel utilizing 1:1 diethyl ether/hexane as the eluant, and the eluant is evaporated at reduced pressure to obtain the product as a yellow solid.

A small sample is recrystallized from diethyl ether/hexane to obtain an analytical sample, m.p. 98°-101°C.

The product is a racemate which may be resolved to obtain the 5R and 5S enantiomers.

Step 12 (Reaction a))

Ethyl (±)-erythro-(E)-3,5-dihydroxy-7-[1'-(4"-fluorophenyl)-3'-(1"-methylethyl)-5'-phenyl-1H-pyrrol-2'-yl]hept-6-enoate (Compound No.1)

(a) 4.32 ml. of 1.0M. tri-n-butylborane/tetrahydrofuran (4.32 mmoles) is added quickly dropwise to a solution of 1.0 g. (2.16 mmoles) of Compound CCXVIII in 25 ml. of dry tetrahydrofuran (distilled from ketyl) stirred at 20°-25°C., air is bubbled through the solution for 1 minute, the solution is stirred at 20°-25°C. for 1 hour and cooled to -78°C., 408 mg. (10.8 mmoles) of sodium borohydride is added with stirring at -78°C., and the reaction mixture is stirred at -50°C. for 16 hours and allowed to slowly warm to -20°C. to complete the reaction, the reaction mixture being stirred under dry nitrogen throughout. The reaction mixture is quenched at -20°C. with 10% hydrochloric acid (to pH 2), allowed to warm to 20°-25°C. and partitioned between diethyl ether and water. The aqueous phase is extracted with diethyl ether, and the organic phases are combined, washed twice with saturated sodium chloride solution, dried over anhydrous magnesium sulfate, filtered and evaporated at reduced pressure to a clear yellow oil. A small amount of isopropanol is added, and the isopropanol is evaporated at reduced pressure to obtain a yellow solid.

(b) The cyclic boron ester product of Part (a) is dissolved in methanol with heating and the methanol is evaporated at reduced pressure, and this procedure is repeated twice more to obtain a white solid which is recrystallized from methylene chloride/hexane to obtain the product as a flocculant white solid, m.p. 144.5-146-5°C.

The product is a mixture of the erythro and threo racemates wherein the ratio of the former to the latter exceeds 9:1, which mixture may be separated by conventional means. The principal product, the erythro racemate, may be resolved into two optically pure enantiomers, the 3R,5S and 3S,5R enantiomers, of which

the former is preferred. The minor product, the threo racemate, may be resolved to obtain the 3R,5R and 3S,5S enantiomers. The use of a non-stereoselective reduction would afford a mixture of all four stereoisomers wherein the ratio of the erythro stereoisomers to the threo stereoisomers ranges from 3:2 to 2:3.

Example 2

Sodium
(±)-erythro-(E)-3,5-dihydroxy-7-[1'-(4"-fluorophenyl)-3'-(1"-methylethyl)-5'-phenyl-1H-pyrrol-2'-yl]hept-6-enoate (Reaction e)) (Compound No. 2)

(Substitutents as Compound No. 1 except $R_{10}$ = Na)

0.1 ml. of 0.5N. sodium hydroxide solution (0.05 mmole) is quickly added dropwise to a suspension of 25 mg. (0.054 mmole) of Compound No. 1 in 5 ml. of absolute ethanol, and the reaction mixture is stirred at 20°-25°C. for 2 hours, diethyl ether is added, and the pale yellow precipitated product is collected, stirred in diethyl ether and collected by filtration m.p. 203-206°C (dec.) (yellows at 145°C).

N.M.R ($CD_3OD$ + $CDCl_3$): 1.21 (m, 6H), 1.60 (m, 2H), 2.29 (m, 2H), 3.12 (m, 1H), 3.95 (m, 1H), 4.20 (m, 1H), 5.40 (dd ($J_1$ + 15 Hz., $J_2$ = 7.5 Hz.), 1H), 6.22 (d (J = 15 Hz.), 1H), 6.30 (s, 1H), 7.08 (m, 9H)

The product is a mixture of the erythro and threo racemates wherein the ratio of the former to the latter exceeds 9:1, which mixture may be separated by conventional means. The principal product, the erythro racemate, may be resolved into two optically pure enantiomers, the 3R,5S and 3S,5R enantiomers, of which the former is preferred. The minor product, the threo racemate, may be resolved to obtain the 3R,5R and 3S,5S enantiomers. The use of a starting material synthesized by using a non-stereoselective reduction in Step 12 of Example 1 would afford a mixture of all four stereoisomers wherein the ratio of the erythro stereoisomers to the threo stereoisomers ranges from 3:2 to 2:3.

Example 3

Ethyl (E)-3,5-dihydroxy-7-[3'-(4"-fluorophenyl)-1'-(1"-methylethyl)-5'-phenyl-1H-pyrrol-2'-yl]hept-6-enoate (Compound No. 3)

(Type IBa, X=(E) CH=CH, $R_9$=H, $R_{10}$=$C_2H_5$, $R_2$=p-fluorophenyl, $R_3$=hydrogen, $R_4$=phenyl, $R_1$=i-propyl)

Step 1 (Reaction AD)

(E)-3-(4'-Fluorophenyl)-1-phenylprop-2-en-1-one (Compound CCXXIII)

52.0 g (433 mmoles) of acetophenone is added to a solution of 21.8 g. (545 mmoles) of sodium hydroxide in a mixture of 196 ml. of water and 122.5 ml. of 95% ethanol stirred at 20°-25°C., the resulting suspension is cooled to 0°-5°C., and 53.74 g. (433 mmoles) of 4-fluorobenzaldehyde is added portionwise, the temperature of the reaction mixture rising to 20°C. during the addition. The solidified reaction mixture is kept at 20°-25°C. for 3 hours and refrigerated for 16 hours. The solid is collected by filtration, rinsed with about 2 l. of water until the rinses are neutral, rinsed with 200 ml. of cold 95% ethanol and recrystallized from 95% ethanol to obtain the product as yellow flakes (66.25 g. (68%)), m.p. 83°-86°C.

The reaction then proceeds analogously to Steps 3 to 11 of Example 1 via the following intermediates:

Ethyl (±)-2-[1'-(4"-fluorophenyl)-3'-oxo-3'-phenylpropyl]-1,3-dithiolane-2-carboxylate, viscous clear brown liquid

Ethyl (±)-2,5-dioxo-3-(4'-fluorophenyl)-5-phenylpentanoate

Ethyl 3-(4'-fluorophenyl-1-(1'-methylethyl)-5-phenyl-1H-pyrrole-2-carboxylate, m.p. 97-100°

3-(4'-Fluorophenyl)-1-(1'-methylethyl)-5-phenyl-1H-pyrrole-2-methanol, white powder

3-(4'-Fluorophenyl)-1-(1'-methylethyl)-5-phenyl-1H-pyrrole-2-carboxaldehyde, m.p. 118-122°

Ethyl (E)-3-[3'-(4"-fluorophenyl)-1'-(1"-methylethyl)-5'-phenyl-1H-pyrrol-2'-yl]prop-2-enoate, m.p. 84-87°

(E)-3-[3'-(4"-Fluorophenyl)-1'-(1"-methylethyl)-5'-phenyl-1H-pyrrol-2'-yl]prop-2-en-1-ol

(E)-3-[3'-(4"-Fluorophenyl)-1'-(1"-methylethyl)-5'-phenyl-1H-pyrrol-2'-yl]prop-2-en-1-al, bright yellow foam

Ethyl (±)-(E)-7-[3'-(4"-fluorophenyl)-1'-(1"-methylethyl)-5'-phenyl-1H-pyrrol-2'-yl]-5-hydroxy-3-oxohept-6-enoate, viscous yellow oil

Ethyl (E)-3,5-dihydroxy-7-[3'-(4"-fluorophenyl)-1'-(1"-methylethyl)-5'-phenyl-1H-pyrrol-2'-yl]hept-6-enoate (Compound No. 3)

(a) 1.18 ml. of 1.0M. tri-n-butylborane/tetrahydrofuran (1.18 mmoles) is quickly added dropwise to a solution of 273.2 mg. (0.59 mmole) of ethyl (±)-(E)-7-[3'-(4"-fluorophenyl)-1'-(1"-methylethyl)-5'-phenyl-1H-pyrrol-2'-yl]-5-hydroxy-3-oxohept-6-enoate in 5 ml. of dry tetryhydrofuran (distilled from ketyl) stirred at 20° - 25°C., air is bubbled into the reaction mixture for 1 minute, the reaction mixture is stirred at 20°-25°C. for 1 hour and cooled to -78°C., 56 mg. (1.475 mmoles) of sodium borohydride is added with stirring at -78°C., the reaction mixture is stirred at -50°C. for 16 hours, an additional 56 mg. (1.475 mmoles) of sodium borohydride is added with stirring at -50°C., and the reaction mixture is allowed to slowly warm to -10°C. and is stirred at -10°C. for 1 hours, the reaction mixture being maintained under dry nitrogen throughout. The reaction mixture is quenched at -10°C. with 10% hydrochloric acid (to pH 2),

warmed to 20°-25°C. and partitioned between diethyl ether and water, and the aqueous phase is extracted with diethyl ether. The diethyl ether phases are combined, washed twice with saturated sodium chloride solution, dried over anhydrous magnesium sulfate, filtered and evaporated at reduced pressure to obtain the cyclic boron ester as a clear yellow liquid.

b) The cyclic boron ester product of Part (a) is dissolved in methanol with gentle heating and the methanol is evaporated at reduced pressure, this process is repeated twice more, and the obtained yellow oil is dissolved in the minimum amount of methylene chloride and flash chromatographed on 150 g. of 230-400 mesh A.S.T.M. silica gel utilizing 80% diethyl ether/hexane and then diethyl ether as the eluants. Evaporation of the eluant yields the product as a clear green oil

N.M.R. ($CDCl_3$): 1.28 (t, 3H), 1.50 (d, 6H), 1.60 (m, 2H), 2.48 (m, 2H), 3.28 (m, 1H), 3.71 (m, 1H), 4.20 (m, 3H), 4.46 (m, 1H), 4.59 (m, 1H), 5.56 (dd ($J_1$ = 15 Hz., $J_2$ = 5 Hz.), 1H), 6.14 (s, 1H), 6.75 (d (J = 15 Hz.), 1H), 7.00 (t, 2H), 7.39 (m, 7H)

The product is a mixture of the erythro and threo racemates wherein the ratio of the former to the latter is about 3:1, which mixture may be separated by conventional means. The principal product, the erythro racemate, may be resolved into two optically pure enantiomers, the 3R,5S and 3S,5R enantiomers, of which the former is preferred. The minor product, the threo racemate, may be resolved to obtain the 3R,5R and 3S,5S enantiomers. The use of a non-stereoselective reduction would afford a mixture of all four stereoisomers wherein the ratio of the erythro stereoisomers to the threo stereoisomers ranges from 3:2 to 2:3.

Example 4

Ethyl
(±)-erythro-(E)-3,5-dihydroxy-7-[3'-(4"-fluorophenyl)-1'-(1"-methylethyl)-5'-phenyl-1H-pyrrol-2'-yl]hept-6-enoate (Compound No. 4)

(Substituents as Compound No. 3)

A mixture of 120.4 mg. (0.26 mmole) of Compound No. 3, 24 mg. (0.39 mmole) of boric acid and 5 ml of isopropanol is stirred at 80°C for 3 hours, cooled to 20° - 25°C and evaporated at reduced pressure to obtain a yellow oil. The yellow oil is dissolved in isopropanol with warming, the isopropanol is evaporated at reduced pressure to obtain a yellow foam, the yellow foam is dissolved in the minimum amount of hot isopropanol, the solution is stored at 0°C. for 16 hours, and the mother liquor is decanted. The residual yellow wax is dissolved in methanol with gentle heating and the methanol is evaporated at reduced pressure and this process is repeated twice to obtain a clear yellow oil. The oil is crystallized from methylene chloride/hexane to obtain the product as a flocculant white solid (29.5 mg.), m.p. 105°-107°C.

The product is a mixture of the erythro and threo racemates wherein the ratio of the former to the latter exceeds 9:1, which mixture may be separated by conventional means. The principal product, the erythro racemate, may be resolved into two optically pure enantiomers, the 3R,5S and 3S,5R enantiomers, of which the former is preferred.

Example 5

Sodium
(±)-erythro-(E)-3,5-dihydroxy-7-[3'-(4"-fluorophenyl)-1'-(1"-methylethyl)-5'-phenyl-1H-pyrrol-2'-yl]hept-6-enoate (Reaction e)) (Compound No. 5)

(Substituents as Compound No. 3 except $R_{10}$=Na)

0.08 ml. of 0.5N. sodium hydroxide solution (0.04 mmole) is added dropwise to a solution of 20 mg. (0.043 mmole) of Compound No. 4 in 2 ml. of absolute ethanol stirred at 20°-25°C., the reaction mixture is stirred at 20°-25°C. for 2 hours, the ethanol is evaporated at reduced pressure, the residue is dissolved in water, and the obtained solution is extracted with diethyl ether and lyophilized for 16 hours to obtain the product as a white powder, m.p. 185°-192°C. (dec.) (changes color at 135°C.)

N.M.R. ($CD_3OD$ ± $CDCl_3$): 1.48 (m, 6H), 1.64 (m, 2H), 2.30 (m, 2H), 4.00 (m, 1H), 4.34 (m, 1H), 4.55 (m, 1H), 5.50 (dd ($J_1$ = 15 Hz., $J_2$ = 7.5 Hz.), 1H), 6.02 (s, 1H), 6.71 (d (J = 15 Hz.), 1H), 7.00 (t (J = 10 Hz.), 2H), 7.38 (m, 7H)

The product is a mixture of the erythro and threo racemates wherein the ratio of the former to the latter exceeds 9:1, which mixture may be separated by conventional means. The principal product, the erythro racemate, may be resolved into two optically pure enantiomers, the 3R,5S and 3S,5R enantiomers, of which the former is preferred. The minor product, the threo racemate, may be resolved to obtain the 3R,5R and 3S,5S enantiomers. The use of a starting material synthesized by using a non-stereoselective reduction in Step 11 of Example 3 would afford a mixture of all four stereoisomers wherein the ratio of the erythro stereoisomers to the threo stereoisomers ranges from 3:2 to 2:3.

Example 6

(±)-erythro-(E)-7-[2-tert.-butyl-4-(4-fluorophenyl)-5-phenyl-fur-3-yl]-3,5-dihydroxy-hept-6-enoic acid, ethyl ester (Compound No. 6)

(Type IDa, $R_1$ = t-butyl, X = (E) CH = CH, $R_9$ = H, $R_{10}$ = $C_2H_5$. $R_2$ = p-fluorophenyl, $R_3$ = phenyl)

Step 1, 2-tert.-butyl-4-(4-fluorophenyl)-5-phenyl-fur-3-yl-methanol

Under a dry nitrogen atmosphere, to a slurry of 1.15 g (30.4 mmol) of lithium aluminium hydride in 50 ml of dry ether at about 0° was added 5.56 g (15.2 mmol) of 4-(4-fluorophenyl)-2-tert.-butyl-5-phenyl-3-furyl-carboxylic acid, ethyl ester in 25 ml of dry ether. The mixture was heated at reflux for one-half hour.

The solution was cooled to 0° C, and quenched with water until a white precipitate persists. The precipitate is filtered off, and washed with ether on the filter plate. The combined washings and the filtrate are dried (over magnesium sulfate) and concentrated to a white solid, which is used directly for the next step.

Step 2, 2-tert.-butyl-4-(4-fluorophenyl)-5-phenyl-3-furylcarboxaldehyde

Under a dry nitrogen atmosphere, to a slurry of 583 mg (0.608 mmol) of TTPRD and 3.56 g (30.4 mmol) of NMO (N-methylmorpholine N-oxide) in 50 ml of acetone was added 4.92 (15.2 mmol) of the alcohol product of Step 1, above, in 10 ml of acetone at room temperature), and the mixture was stirred (at RT) for 2 hours.

The mixture was then concentrated and the resulting black residue taken up in ether. The ether solution was then washed three times with 10% hydrochloric acid, once with saturated aq. sodium bicarbonate, then with brine and dried (over anh. magnesium sulfate and concentrated to a black oil. Upon standing for about 18 hours in the freezer the oil had formed a solid. The solid was recrystallized from ethanol to give a first crop of a grey powder, and the mother liquor further processed.

Additional product was obtained from the mother liquor by chromatography (silica gel, 25% ether/hexane).

Step 3, 3-[2-tert.-butyl-4-(4-fluorophenyl)-5-phenylfur-3-yl]-2-propenal (trans)

Under an atmosphere of dry nitrogen, 1.56 g (9.3 mmol, 1.10 ml) of cis-bromoethoxy ethylene was added to about 20 ml of dry THF and cooled to -78°. 11.0 ml (18.6 mmol) of tert.-butyl lithium was cannulated into a dropping funnel and then added dropwise at -78°. The mixture was stirred for 2 hrs. at at -78°.

2.0g (6.2 mmol) of the aldehyde product of Step 2, above in about 5 ml of dry THF was added dropwise at -78° and the mixture stirred for 15 minutes (TLC indicated no starting material present). The mixture was then quenched with about 5 ml of saturated aq. ammonium chloride, stirred until the mixture was homogenous, and then extracted twice with 25 ml portions of ether. The combined ether extracts were washed once with brine, dried over anhyd. magnesium sulfate and concentrated to a brown oil. The oil was dissolved in 10% aqueous THF to which toluene sulfonic acid (TSOH) had been added. After stirring for 15 minutes the solution was diluted with saturated aq. sodium bicarbonate, extracted twice with 25 ml portions of ether and the combined ether extracts were washed once with brine, dried over anhydrous magnesium sulfate and concentrated to a brown oil. The oil was dissolved in about 3 ml of ethanol and the solution stored in a freezer. The product of this step was isolated from the reaction mixture as light yellow crystals.

Additional product was recovered by flash chromatography 2:1 (hexane/ether).

Step 4, 7-[2-tert.-butyl-4-(4-fluorophenyl)-5-phenylfur-3-yl]-5-hydroxy-3-oxo-hept-6-enoic acid, ethyl ester

Under a dry nitrogen atmosphere, 398 mg of 60% sodium hydride in paraffin oil (9.96 mmol) is charged to a flask, washed twice with hexane, and then suspended in about 150 ml of dry THF. The mixture is cooled to about -15° (using ice/methanol) and 1.25g (9.57 mmol) of neat ethyl acetoacetate added thereto, dropwise. The mixture is stirred until it clears (about 15 minutes). 6.30 ml of 1.55 m n-butyl lithium (in hexane) (9.76 mmol) is added by syringe at -15°C and the mixture stirred for 15 minutes.

1.335g (3.83 mmol) of the aldehyde product obtainable by the method of Step 3, above, dissolved in about 25 ml of dry THF is added, dropwise at -15°C. The mixture is stirred for 15 minutes, then quenched with water.

The quenched mixture is extracted twice with portions of ether. The ether extracts are combined and washed once with brine, dried over anhydrous magnesium sulfate and concentrated to a yellow oil (crude product of this step). The oil is flash chromatographed (3:2 ether/hexane) to obtain the refined title product of this step as a clear oil.

Step 5, Butyl borane complex intermediate

Under a dry nitrogen atmosphere, 890 mg (1.88 mmol) of the oxoheptenoate of Step 4, above, was dissolved in 20 ml of dry THF and 3.75 ml of 1.0M tri-n-butyl borane in THF (3.75 mmol) was added at once. Air was bubbled through the solution for 1 minute and this was allowed to stir (at RT) for 1 hour (yellow mixture). The reaction mixture was cooled to about -55° (using a cryogenic cooler) and 213 mg (5.63 mmol) of sodium borohydride was added at once. The mixture was stirred at -55° for about 65 hours.

The reaction was quenched with about 5 ml of water and extracted twice with 25 ml portions of ether. The combined ether extracts were washed once with brine, dried over anhyd. magnesium sulfate and concentrated to crude product of this step as a yellow oil.

About 3 ml of isopropanol was added to the oil (crude product) and held in a freezer for about 50 hrs. The refined product was isolated as a white powder (m.p. 55-58°), for use in the next step.

Step 6, (±)-erythro-(E)-7-[2−tert.-butyl-4-(4-fluorophenyl)-5-phenyl-fur-3-yl]3,5-dihydroxy-hept-6-enoic acid, ethyl ester

379 mg (0.69 mmol) of the refined boron intermediate prepared in Step 5, above, was dissolved in methanol (requiring slight heating) and the solution concentrated on a rotary evaporator (repeating 5 times) to obtain a residue.

The residue was purified by column chromatography (15%) ether-methylene chloride) and concentrated to give 281 mg of final product as a clear oil. The oil was taken up in pentane-ether and crystallized to give the refined title product as a white powder. (m.p. 94-95° C; 96% erythro diol).

EXAMPLE 7

7-[4-(4-fluorophenyl)-2-isopropyl-5-phenyl-fur-3-yl]-3,5-dihydroxy-hept-6-enoic acid, ethyl ester (Compound No. 7)

(Type IDa, $R_1$=i-propyl, X=(E) CH=CH, $R_9$=H, $R_{10}$=$C_2H_5$, $R_2$=p-fluorophenyl, $R_3$=phenyl)

Step 1, 3-[4-(4-fluorophenyl)-2-isopropyl-5-phenyl-fur-3-yl]-prop-2-enoic acid, ethyl ester

Under essentially moisture-free conditions, 740 mg of 60% sodium hydride in paraffin oil (18.9 mmol) is charged to a vessel and washed three times with hexane. Excess hexane is removed under a stream of dry nitrogen gas. About 25 ml of dry THF is introduced and then 3.95g (3.3 ml; 17.6 mmol) of triethyl phosphonoacetate. (The first 3-5 drops being added at RT, while the remainder is added at 0°). The mixture is stirred at RT until a clear solution is obtained (about 15 to 30 minutes). 2.71g (8.8 mmol) of 4-(4-fluorophenyl)-2-isopropyl-5-phenyl-3-furylcarboxaldehyde (prepared analogously to Example 6, Steps 1 and 2) in about 15 ml of dry THF is added, dropwise, at RT. The mixture is stirred for about 24 hours.

The reaction mixture is then concentrated, taken up in ether, the solution washed twice with brine, dried over anhydrous magnesium sulfate, and concentrated. Slow crystallization of the title product of this step occurs on standing in a freezer. The solids are recrystallized from ethanol, m.p. 116-118°.

Step 2, 3-[4-(4-fluorophenyl)-2-isopropyl-5-phenylfur-3-yl]prop-2-enol

Under a nitrogen atmosphere, 2.57 g (6.8 mmol) of the ester product of Step 1, above, is dissolved in 100 ml of dry ether, and cooled to -78°. 18 ml of 1.5 M DiBAH (27 mmol) is added dropwise. The mixture is stirred at -78° for 15 minutes. The mixture is quenched with excess water (about 5 ml), and stirred at RT for about 45 minutes (until gel separates as a precipitate). The mixture is filtered over a bed of sand, the precipitate washed with ether, the filtrate dried over magnesium sulfate and concentrated to obtain crude title product of this step as a residue, in which form it can be used for the next step (Step 3), described below.

Step 3, 3-[4-(4-fluorophenyl)-2-isopropyl-5-phenylfur-3-yl]-2-propenal

Under a nitrogen atmosphere, to a suspension of 4 mg (0.004 mmol) of TTPRD and 30.5 mg (0.26 mmol) of NMO in 15 ml of acetone was added 45 mg (0.13 mmol) of the alcohol product of Step 2, obtained as described above, in 3 ml acetone, at RT. The mixture was stirred for about 18 hours. TLC indicated incomplete consumption of starting material. An additional equivalent of NMO (one half the original amount) and 0.03 equivalent (equal to original amount) of TTPRD was added, and the mixture stirred at RT for one hour.

The mixture was concentrated, to a residue. The residue was taken up in ether, washed three times with 10% hydrochloric acid, sat. aq. sodium bicarbonate and then brine, dried over magnesium sulfate, and concentrated to a brown oil. The oil was passed through a plug of silica (using 25% ether, methylene chloride) and the filtrates concentrated to a yellow solid. The solid was recrystallized from hexane to give the title product of this step as yellow granules, m.p. 124-125°.

Proceeding analogously to Steps 4, 5 and 6 of Example 6 the title product is obtained m.p. 98-100° (erythro:threo=87:13)

Example 8

(E)-7-[4-(4-fluorophenyl)-2-isopropyl-5-phenylthien-3-yl]-3,5-dihydroxy-hept-6-enoic acid, ethyl ester (Compound No.8)

(Type IAa substituents as for Compound No. 7)

Step 1, 4-(4-fluorophenyl)-2-isopropyl-5-phenyl-3-thiophenemethanol

150 ml of a 1.0 m solution of lithium aluminum hydride in THF was diluted with 400 ml of dry THF. To this was added dropwise a solution of 40g of the title ester product of Preparation 3, below, in 100 ml of THF. After the addition was complete, the reaction was stirred at R.T. for 5 hours. Approximately 10-15 ml of saturated aq. sodium sulfate solution was added dropwise until a thick precipitate formed. The mixture was diluted with MTBE and the solids were filtered and washed well with MTBE. The filtrate was evaporated under reduced pressure to give 18.2 g of crude title product of this step, which was refined by chromatographing on a waters prep-500 apparatus using methylene chloride as eluate, to obtain the refined title product of this step, m.p. = 140-143°.

Step 2, 4-(4-fluorophenyl)-2-isopropyl-5-phenylthiophenecarboxaldehyde

To a solution of 1.92 g of oxalyl chloride in 50 ml of methylene chloride at -78° was added dropwise a solution of 2.22 g of dimethylsulfoxide in 25 ml methylene chloride. After stirring at -78° for 10 minutes, a solution of 4.0 g of the title methanol product of Step 1, above, in 30 ml of methylene chloride was added dropwise. The mixture was stirred at -78° for 1 hour. Then 2.5 g of triethylamine was added. The mixture was allowed to warm to R.T., then was poured into water. The organic phase was separated and the solvent was removed under reduced pressure. The residue was dissolved in MTBE and washed with water. The organic solution was dried, and the solvent was removed under reduced pressure to give the title product of this step. A sample of which was triturated with pentane to give refined product. M.P. 116-120°.

Step 3, 3-[4-(4-fluorophenyl)-2-isopropyl-5-phenythien-3-yl]-2-propenal (trans)

To a solution of 2.4 g of cis-2-bromo-ethoxyethylene in 75 ml of THF at -78°, was added dropwise 2.0 g of t-butyllithium (2.2. m in pentane). After stirring at -78° for 2 hours, a solution of 3.5 g of the aldehyde product of Step 2, above, in 30 ml of THF was added dropwise. The reaction was stirred at -78° for 10 minutes, then was quenched by the addition of saturated aq. ammonium chloride solution. The mixture was extracted once with MTBE and then once with methylene chloride. The organic solutions were combined, dried over anhyd. magnesium sulfate and concentrated under reduced pressure to obtain a residue. The residue was dissolved in 100 ml of THF/water (90:10). p-toluenesulfonic acid (0.7 g) was added and then the mixture was stirred at room temperature for 1 hour. The reaction mixture was poured into 10% aq. sodium bicarbonate solution and the organic material was extracted with MTBE. The solution was dried and the solvent removed under reduced pressure to give the title product of this step for use in the next step ( 4 ). An analytical sample was crystallized from hexane; m.p. 138-140°;

Step 4, 7-[4-(4-fluorophenyl)-2-isopropyl-5-phenylthien-3-yl]-5-hydroxy-3-oxo-hept-6-enoic acid, ethyl ester.

To a solution of 2.6 g of diisopropylamine in 30 ml of THF at 0° was added 1.67 g of n-butyllithium (1.16 m in hexane). Then a solution of 1.7g of ethyl acetoacetate in 10 ml of THF was added dropwise. After stirring at 0° for 1 hour, the mixture was cooled to -20°, then a solution of 3.3 g of the title product of Step 3, above, in 20 ml of THF was added dropwise. The reaction was stirred at -20° for 1 hour. Then was quenched with saturated aqueous ammonium chloride solution. The mixture was extracted with MTBE and then methylene chloride and the organic solutions were combined and dried, and the solvent evaporated under reduced pressure to give the crude title product of this step as an oil. This was used without further purification in the next step (Step 5).

Step 5, (E)-7-[4-(4-fluorophenyl)-2-isopropyl-5-phenylthien-3-yl]-3,5-dihydroxy-hept-6-enoic acid, ethyl ester

To a solution of 5.5 g of the title product of Step 4, above, in 100 ml of THF was added 12 ml of a 1.0 m solution of triethylborane in THF. After stirring at R.T. for 3 hours, the mixture was cooled to -78°, then 450 mg of sodium borohydride was added. The mixture was stirred at -78° for 24 hours. The reaction was then quenched with saturated aq. ammonium chloride solution, and extracted twice with MTBE. The extracts were combined and solvent was removed under reduced pressure to obtain a residue. The residue dissolved in 100 ml of methanol. After stirring at R.T. for 24 hours, the solvent was removed under reduced pressure and the resulting residue was flash chromatographed using 1% methanol/methylene chloride to elute the product, yielding the title product of this example. (HPLC indicated an erythro:threo mixture of 82.3:17.7 Recrystallization of the product from cyclohexane increased the ratio to 88.3:11.7; m.p. = 123-125°.

(E)-7-(4-phenyl-2-isopropyl-5-phenylthien-3-yl)-3,5-dihydroxy-hept-6-enoic acid, ethyl ester, M.P. 105-6° (Compound No. 9)

(Type IAa substituents as compound No. 8 except $R_2$ = phenyl). Obtain analogously to Examples 6,7 or 8 above.

Example 9

Sodium (±)-erythro-(E)-7-[4-(4-fluorophenyl)-2-isopropyl-5-phenylthien-3-yl]-3,5-dihydroxy-hept-6-enoate (Compound No. 10)

(Type IAa substituents as Compound No. 8 except $R_{10}$ = Na)

To a solution of 482 mg of Compound No. 8 in 25 ml of ethanol was added 1.0 ml of 1.0 N NaOH. After stirring at 50° for 2 hours, the solvent was removed under reduced pressure. The residue was dissolved in water and the solution was washed with MTBE. The aqueous phase was separated and lyophilized to give the title product of this example. M.P. 247-250° (dec.).

Example 10

The following compounds can be prepared analogously to Example 2, 5 or 9 from the corresponding Compounds 6, 7 and 9.

Sodium erythro(E)-7-[2-tert.butyl-4-(4-fluorophenyl)-5-phenyl-fur-3-yl]-3,5-dihydroxyhept-5-enoate (m.p. 221-227°); Compound No. 11

Sodium erythro(E)-7-[4-(4-fluorophenyl-2-isopropyl-5-phenyl-fur-3-yl]-hept-6-enoate (m.p. 221-229°); Compound No. 12

Sodium erythro(E)-7-(4-phenyl-2-isopropyl-5-phenylthien-3-yl)-3,5-dihydroxyhept-6-enoate (m.p. over 220°, dec.); Compound No. 13

Example 11

Adapting the procedure of Examples 1 to 9 as appropriate the following compounds IAa are obtained in which X is (E)CH=CH, $R_1$ is isopropyl, $R_9$ is hydrogen and $R_{10}$ is ethyl (the erythro isomer dominating by at least 4:1); (in the Table, Me = methyl, Et = ethyl, ip = isopropyl, cp = cyclopentyl, and ch = cyclohexyl, d = decomposition, ph = phenyl) :

| Compound Nos. | $R_2$ | $R_3$ | M.P.° etc. of: Ester | Na-Salt |
|---|---|---|---|---|
| 14,15 | 4-F-ph | 4-Me-ph | 121-2 | 215-20(d) |
| 16,17 | 4-F-ph | 4-Cl-ph | 130-1 | 210-6(d) |
| 18,19 | 4-F-ph | ip | 93.5-94 | 220-230(d) |
| 20,21 | 4-F-ph | 4-MeO-ph | 97-9 | 116-121(d) |
| 22,23 | 3-F-ph | ph | 103-4 | |
| 24,25 | ph | Me | oil | 204-7(d) |
| 26,27 | ch | ph | oil | 230-5 |
| 28,29 | 2-F-ph | ph | oil | 208-10 |
| 30,31 | cp | ph | oil | 220-5(d) |
| 32,33 | 4-MeOph | ph | oil | 211-6 |
| 34,35 | 3-$CF_3$-ph | ph | gum | d |
| 36,37 | 4-ph-ph | ph | 61-4 | 235(d) |

(Esters even numbers, sodium salts odd numbers).

Adapting the procedure of Example 2, 5 or 9 to treat the ethyl esters of Example 11, above, the corresponding sodium salts thereof are accordingly obtained.

Example 12

Adapting the procedure of Examples 1 to 9, the following compounds IAa are obtained in which $R_1$ is isopropyl and $R_9$ is hydrogen, (the erythro isomer predominating about 4:1); (in the table Et = ethyl) cp = cyclopentyl, dm = $-CH=C(CH_3)_2$ and VN = (E) -CH=CH-

| Compound No. | $R_2$ | $R_3$ | $R_{10}$ | X |
|---|---|---|---|---|
| 38 | 4-F-ph | cp | Et | VN |
| 39 | 4-F-ph | cp | Na | VN |
| 40 | 4-F-ph | ph | Et | $-(CH_2)_2-$ |
| 41 | 4-F-ph | ph | Na | $-(CH_2)_2-$ |
| 42 | 4-F-ph | dm | Et | VN |
| 43 | 4-F-ph | dm | Na | VN |

Starting materials for use in Examples 6 to 11 are prepared e.g. as follows:

Preparation 1

2-t-butyl-4-(4-fluorophenyl)-5-phenyl-3-furylcarboxylic acid, ethyl ester

Step 1 : α-Bromo-α-(p-fluorophenyl)-acetophenone

At room temperature, to 20 g (0.093 mol) of α-(p-fluorophenyl)-acetophenone in 200 ml of chloroform, are added a few drops of a charge of 4.8 ml of bromine (0.93 mol) (a deep orange-color results). The resultant

solution is heated to initiate reaction (the solution turns yellow), and then cooled to 0° C, and the balance of the 4.8 ml of bromine is added dropwise. The reaction mixture is stirred at room temperature for about 16 hours.

The orange solution is washed three times with saturated aqueous sodium bicarbonate, then once with brine (saturated aqueous sodium chloride), dried, and concentrated to an orange oil, which partially crystallizes.

The product is refined by recrystallization from ethanol yielding refined title product of this step as a white powder.

Step 2 : Ethyl 2-(α-benzoyl-4-fluorobenzyl)-3-oxo-4,4-dimethylpentanoate

Into a vessel, under a dry nitrogen atmosphere 1.05g (26.3m mol) of sodium hydride (60%) in paraffin oil was washed twice with hexane, then suspended in 50 ml of dry THF. 4.3g (25.1m mol) of ethyl 4,4-dimethyl-3-oxopentanoate was added dropwise at 0°C, the reaction mixture was stirred for 15 minutes (resulting in a clear solution). 7.0g (23.9m mol) of the bromo product of step 1 of this preparation in 20 ml of THF was added dropwise at 0°C to the mixture in the flask. The resulting mixture was stirred at room temperature for about 21 hours.

The mixture was then concentrated to obtain a residue. The residue was then taken up in ether, washed twice with saturated aq. sodium bicarbonate solution and then once with brine, dried (over anhyd. magnesium sulfate) and concentrated to crude title product as a yellow oil, which was used directly in the next step (3).

Step 3: 2-tert-butyl-4-(4-fluorophenyl)-5-phenyl-3-furylcarboxylic acid, ethyl ester

The crude product of step 2 of this preparation was dissolved in 100 ml. of toluene to which 9 ml (about 3 equivalents) of $BF_3 \bullet O(C_2H_5)_2$ was added and the mixture refluxed for 1¼ hours. The reaction mixture was then poured into 150 ml of sat. aq. sodium bicarbonate and extracted twice with 100 ml portions of ether. The combined ether extracts were washed once with brine, dried (over anhyd. magnesium sulfate) and concentrated. The title product of this preparation (brown solid) was recrystallized from ethanol, M.P. 93-94°.

Preparation 2

Repeating the procedure of preparation 1 but in step 2, using in place of the ethyl 4,4-dimethyl-3-oxo-pentanoate an approximately equivalent amount of ethyl 4-methyl-3-oxo-pentanoate there is accordingly obtained the 2-isopropyl analog of the product of that preparation (M.P. 69-70°).

Preparation 3

4-(4-Fluorophenyl)-2-isopropyl-5-phenyl-3-thiophenecarboxylic acid, ethyl ester

Step 1 : α-(4-Fluorophenyl)-β-mercapto-benzene ethanol

To a solution of 12.4g (0.1 mole) of benzyl mercaptan (ie, benzenemethanethiol) in 150 ml of THF at 0°C under moisture-free conditions, was added, dropwise, 14g (0.22 mole) of n-butyllithium (1.6m in hexane). After stirring at 0°C for 4 hours (orange precipitate forms), the mixture was cooled to -25°. To this mixture was added a solution of 12.4g (0.1 mole) of p-fluorobenzaldehyde in 100 ml of THF, dropwise. After stirring at -25°C for 1 hour, a yellow solution occurs. The reaction was quenched with saturated aqueous ammonium chloride and the organic material was extracted once with MTBE and then once with methylene chloride. The organic solutions were combined and dried, and the solvent was removed under reduced pressure to give the title product of this step as an oil (80:20 mixture of isomers), which was used in the next step without any further purification.

Step 2, 2-diethoxyphosphinyl-4-methyl-pent-3-enoic acid, ethyl ester

A mixture of 22.4g (0.1 mole) of triethylphosphonoacetate, 40g (0.56 mole) of isobutyraldehyde, 3 ml of acetic acid, and 0.6g of piperidine in 200 ml of benzene was refluxed in a Dean Stark apparatus for 48 hours. The solvent was removed under reduced pressure and the resulting oil was distilled at 0.33 mm, b.p. 110-120° (product is composed of an isomeric mixture ratio 85:15).

Step 3, 2-(diethoxyphosphinyl)-3-[2-(4-fluorophenyl)-2-hydroxy-1-phenylethyl]-thio-4-methyl-pentanoic acid, ethyl ester

To a solution of 24.8g (0.1 mole) of the mercapto product of step 1, above, and 28.4g (0.1 mole) of the ester product of step 2, above, in 300 ml of THF was added 11 g (0.11 mole) of triethylamine. The mixture was then stirred at room temperature for 4 hours. Then the reaction mixture was poured into water and the organic material was extracted once with MTBE and once with methylene chloride. The organic solutions were combined and dried, and the solvent was removed under reduced pressure to give the title product of this step, as a oil (mixture of isomers). This was used in the next step (4) without further purification.

Step 4, 2-(diethoxyphosphinyl)-3-[2-(4-fluorophenyl)-2-oxo-1-phenylethyl]-thio-4-methyl-pentanoic acid, ethyl ester

To a solution of 12.2g (0.096 mole) of oxalyl chloride in 1 litre of methylene chloride at -68°C was added dropwise a solution of 15g (0.194 mole) of DMSO in 40 ml of methylene chloride. After stirring at -65° for 5

minutes, a solution of 42g (0.08 mole) of the product of step 3 in 200 ml of methylene chloride was added dropwise. Stir at -65°C for 1 hour. Then 40g (0.4 moles) of triethylamine was added. After allowing the mixture to warm to RT, the mixture was poured into water. The organic phase was separated and the solvent was removed under reduced pressure. The residue was dissolved in MTBE and the solution was washed with water. The organic phase was dried and the solvent was removed under reduced pressure to give the crude title product of this step as an oil.

Step 5, 4-(4-fluorophenyl)-2,5-dihydro-2-isopropyl-5-phenyl-3-thiophenecarboxylic acid, ethyl ester

Under essentially moisture-free conditions, to a solution of 8.1g of diisopropylamine in 500 ml of THF at 0° was added 5.25g of n-butyllithium (1.6 m in hexane). After stirring at 0° for 5 minutes, a solution of 45g of the carbonyl product of step 4, above, in 100 ml THF was added rapidly after stirring at room temperature for 1 hour. The mixture was quenched with saturated aqueous ammonium chloride.

The mixture was extracted with MTBE and the organic phase was dried. The solvent was removed under pressure to give the title product of this step as an oil, which was used in the next step (6) without further purification.

Step 6, 4-(4-fluorophenyl)-2-isopropyl-5-phenyl-3-thiophenecarboxylic acid, ethyl ester

To a suspension of 25 g of 2,3 dichloro-5,6-dicyanobenzoquinone in 1 liter of methylene chloride at room temperature was added slowly a solution of 40g of the dihydrothiophene carboxylate of Step 5, above, in 125 ml of methylene chloride. The mixture was stirred at room temperature for 24 hours, filtered, and the filtrate was washed with 10% aqueous sodium bicarbonate solution. The organic phase was separated and the solvent was removed under reduced pressure. The residue was filtered through a pad of silica gel to remove any polar material. Evaporation of the solvent gave the title product of this preparation.

PREPARATION 4

2-Isopropyl-5-(4-chlorophenyl)-4-(4-fluorophenyl)-3-thienylcarboxylic acid, ethyl ester

Step 1, 4-Fluoro-[bis-(methylthio)methylbenzene (may also be called α,α-di-(methylmercapto)p-fluorotoluene)

To a mixture of 196 g (1.56 mol) of p-fluorobenzaldehyde and 100 g (3.12 mol) of methanethiol in 1200 ml of ether in a 1 liter 2-neck flask equipped with a dry ice condenser was added, dropwise, 32.16 ml of $BF_3 \bullet O(C_2H_5)_2$ at 0°C. The mixture was stirred at 0°C for 4 hours, then treated with 10% aqueous sodium hydroxide solution and extracted with ether. After drying (over anhydrous magnesium sulfate) and concentration, the product was distilled under reduced pressure to yield the title product of this step; b.p. 120-122°C.

Step 2 β,β-bis(methylthio)-α-(p-chlorophenyl)p-fluorobenzeneethanol (may also be called 1-(4-chlorophenyl)-2-(4-fluorophenyl)-2,2-di-(methylmercapto)ethanol)

To a stirred solution of 25.0 g (135 mmol) of the dithioacetal product of step 2, above in 200 ml of dry THF at -78° under nitrogen was added 91 ml (140 mmol) of n-butyllithium (1.55 M, in hexane solution). After two hours, 18.7 g (140 mmol) of p-chlorobenzaldehyde was added and the mixture was stirred for another hour. The reaction mixture was poured into saturated aqueous ammonium chloride solution, extracted with ether, washed with water, dried over magnesium sulfate, filtered, and concentrated to give 40 g of the crude title product of this step.

Purification by liquid chromatography (Waters Prep 500) gave the refined title product of this step as an oil.

Step 3 α-(4-chlorophenyl)-α-hydroxy-4-fluoroacetophenone

To a mixture of 27g (78 mmol) of the hydroxydithiolketal product of Step 2, above and 33.8g (156 mmol) of mercuric oxide (red) in 240 ml of 80% THF was added dropwise 40 ml of $BF_3 \bullet O(C_2H_5)_2$. The mixture was stirred at 0°C for 2 hours, then washed with water, 10% aqueous sodium bicarbonate, then brine and dried over anhydrous magnesium sulfate, and evaporated to dryness to obtain crude title product of this step. Purification by prep LC (Waters Prep 500) on silica gel using hexane-methylene chloride (1:1) gave the refined title product of this step.

Step 4 α-bromo-α-(4-chlorophenyl)-4-fluoroacetophenone

To a mixture of 14.0 g (53 mmol) of the hydroxyketone product of Step 3, above, in 300 ml of THF was added 27.7 g (106 mmol) of carbon tetrabromide, followed by 35 g (106 mmol) of triphenylphosphine. The mixture was stirred at room temperature for 6 hours and filtered. After removal of solvent by evaporation, hexane was added to the residue and the resulting mixture was filtered. The filtrate was concentrated to dryness to obtain crude title product of this step, which was purified by chromatography (Waters Prep 500) yielding the refined title product of this step.

Step 5 α-(4-fluorobenzoyl)-4-chlorobenzyl ester of ethanethioic acid (may also be called 1-fluorobenzoyl-1-(4-chlorophenyl)-methylthio acetate)

To a stirred solution of 16g (49 mmol) of the bromoketone product of Step 4, above, in 150 ml of absolute ethanol was added 8.3g (73.5 mmol) of potassium thioacetate and the suspension was stirred at room temperature for about 16 hours. After removal of the solid by filtration, the solution was washed with water, dried over anhydrous magnesium sulfate, and evaporated to give crude title product of this step as an oil which was refined by chromatographing on silica gel using ethyl acetate-hexane (1:9); to yield: the refined title product of this step.

Step 6 α-mercapto-α-(4-chlorophenyl)-methyl-4-fluoroacetophenone (may also be called 1-(p-chlorophenyl)-1-(p-fluorobenzoyl)-methylmercaptan)

A stirred solution of 9.0 g (31.07 mmol) of the thioacetate product of Step 5, above, in 100 ml of methanol was treated with 2.5 ml of concentrated hydrochloric acid at 0° C. The reaction mixture was heated at 60° C for 18 hours and concentrated to obtain a residue. The residue was then dissolved in ether and washed with water and then brine. The ethereal solution was dried over anhyd. magnesium sulfate and evaporated to dryness to obtain crude title product of this step. Chromatographing the crude product on silica gel using ethyl acetate-hexane yielded the refined title product of this step.

Step 7, 2-isopropyl-5-(4-chlorophenyl)-4-(4-fluorophenyl)-2,5-dihydro-3-thiophenecarboxylic acid, ethyl ester.

To a stirred solution of 1.73g (17.1 mmol) of diisopropylamine in 20 ml of dry THF under nitrogen at 0°C was added 11 ml (17.1 mmol) of n-butyllithium. After 15 minutes, 4.0g (14.2 mmol) of the mercaptoketone product of Step 6, above, was added dropwise, and the mixture was stirred at -78° for 30 minutes. 4.5g (14.2 mmol) of ethyl 2-diethoxyphosphinyl-4-methyl-pent-3-enoic acid, (product of Step 2 of Preparation 3, above) was added, and the mixture stirred for 2 hours at -78°. The cooling bath was removed and the solution was allowed to warm to 0° and stirred for two more hours. The mixture was then poured into saturated aqueous ammonium chloride and extracted with ether. The extracts were washed with water, dried over anhydrous magnesium sulfate, filtered, and concentrated to give the crude title product of this step. The crude product was chromatographed on silica gel to yield the refined title product of this step.

Step 8, 2-Isopropyl-5-(4-chlorophenyl)-4-(4-fluorophenyl)-3-thienylcarboxylic acid, ethyl ester

Analogous to Preparation 3, Step 6.

Preparation 5

Adopting the procedure of Preparation 3, the following compounds are analogously obtained, in which $R_1$ is isopropyl and $R_{10}$ is ethyl (in the table: ph = phenyl, Ch = cyclohexyl Cp = cyclopentyl and Me = methyl):

| Preparation No. | $R_2$ | $R_3$ |
|---|---|---|
| 5 | ph | ph |
| 6 | 3-F-ph | ph |
| 7 | Ch | ph |
| 8 | 2-F-ph | ph |
| 9 | Cp | ph |
| 10 | 4-MeOph | ph |
| 11 | 3-$CF_3$-ph | ph |
| 12 | 4-pH-ph | ph |
| 13 | 4-F-ph | 4-MeO-ph |
| 14 | 4-F-ph | 4-Me-ph |

Adopting the procedure of Preparation 4, the compounds of Preparations 5 to 14 above, plus the following compounds may be analogously obtained, in which $R_1$ is isopropyl and $R_{10}$ is ethyl (in the table ph = phenyl; Me = methyl; and ip = isopropyl);

| Preparation No. | $R_2$ | $R_3$ |
|---|---|---|
| 15 | 4-F-ph | ip |
| 16 | ph | Me |

Characterizing Data

The following data characterizes various compounds identified above.

Unless noted otherwise, figures are for NMR run in $CDCl_3$ and run at 200 Hz; s = single, d = doublet, t = triplet, m = multiple and q = quartet. DMSO is dimethyl sulfoxide. HPLC is high pressure liquid chromatography.

Compound No. 10

NMR (DMSO-$d_6$ 40°C)

7.42 - 7.01 (m, 9H), 6.19 (d, 1H), 5.40 (dd, 1H), 4.11 (m, 1H), 3.66 (m, 1H), 3.40 (m, 1H), 2.12 - 1.75 (m, 2H), 1.60 - 1.15 (m, 2H), 1.32 (d, 6H), OH protons fall in with $H_2O$.

Compound No. 11

NMR ($CD_3OD$ ) 1.3 - 1.6 (m, 2H), 1.45 (s, 9H), 2.2 (m, 2H), 3.8 (m, 1H), 4.2 (m, 1H), 5.1 (dd, J = 7.16 Hz, 1H), 6.6 (dd. J = 1.16 Hz, 1H) 7.1 - 7.3 (m, 9H).

Compound No. 12

NMR ($CD_3OD$ )

1.33 (d, J = 7, 6H) 1.4 - 1.7 (m, 2H), 2.2 (m, 2H), 3.9 (m, 1H), 4.2 (m, 1H), 5.4 (dd, J = 7.16, 1H), 6.3 (dd, J = 1.16, 1H), 7.1 - 7.4 (m, 9H).

Compound No. 13

NMR (DMSO - $d_6$)

7 - 7.4 (m, 10H), 6.2 (d, 1H), 5.38 (dd, 1H), 4.1 (m, 1H), 3.6 (m, 1H), 1.75 - 2.1 (m, 2H), 1.34 (d, 6H), 1 - 1.5 (m, 2H).

Compound No. 15

NMR (DMSO - $d_6$)

6.9 - 7.2 (m, 8H), 6.12 (d, 1H), 5.35 (dd, 1H) 4.07 (m, 1H), 3.57 (m, 1H), 2.22 (s 3H), 1.75 - 2.1 (m, 2H), 1.3 (d, 6H), 1.2 - 1.5 (m, 2H).

Compound No. 17

7.06 (m, 8H), 6.40 (d, 1H), 5.40 (dd. 1H), 4.02 (m, 1H), 3.52 (q, 1H), 2.42 (m, 2H), 1.56 (q, 2H), 1.42 (d, 6H).

Compound No. 19

NMR (DMSO-$d_6$)

7.1 - 7.3 (m, 4H), 6.10 (d, 1H), 5.20 (dd, 1H), 4.82 (d, 1H), 4.00 (q, 1H), 3.55 (m, 1H), 2.92 (m, 1H), 1.7 - 2.05 (m, 2H), 1.25 (d, 6H), 1.12 (d, 6H), 1.2 - 1.5 (m, 2H).

Compound No. 21

(DMSO-$d_6$)

7.05 - 7.2 (m, 4H), 7.0 (d, 2H), 6.75 (d, 2H), 6.12 (d, 1H), 5.32 (dd, 1H), 4.06 (m, H), 3.7 (s, 3H), 1.7 - 2.1 (m, 2H), 1.3 (d, 6H), 1.15 - 1.5 (m, 2H).

Compound No. 24

7.42 (m, 3H), 7.18 (q, 2H), 6.42 (d, 1H), 5.37 (dd, 1H), 4.29 (m, 1H), 4.18 (q, 2H), 3.38 (m, 1H), 2.42 (dd, 2H), 1.62 (m, 2H), 1.32 (d, 6H), 1.22 (m, 3H).

Compound No. 25

1R ($CHCl_3$): 3505, 1720, 1195 $cm^{-1}$;

NMR ($CDCl_3$): (Major isomer):

7.42 (m. 5H), 6.65 (dd, 1H), 5.70 (dd, 1H), 4.61 (m. 1H). 4.37 (m, 1H), 4.16 (q, 2H), 3.77 (d, 1H), 3.36 (m, 1H), 3.24 (d, 1H). 2.72 (m, 1H), 2.55 (d, 2H). 2.1 - 1.7 (m, 2H), 1.63 (m, 10H), 1.31 (d, 6H), 1.30 (t, 3H).

Compound No. 26

IR ($CHCl_3$): 3505, 1720, 1195 $cm^{-1}$;

NMR: (major isomer):

7.42 (m, 5H), 6.65 (dd, 1H), 5.70 (dd. 1H), 4.61 (m, 1H), 4.37 (m, 1H), 4.16 (q, 2H), 3.77 (d, 1H), 3.36 (m, 1H), 3.24

(d, 1H), 2.72 (m, 1H), 2.55 (d, 2H), 2.1 - 1.7 (m, 2H), 1.63 (m, 10H), 1.31 (d, 6H), 1.30 (t, 3H); HPLC erythro:threo = 89.2 : 10.8

Compound No. 27
NMR (DMSO-$d_6$)
7.48 - 7.25 (m, 5H), 6.48 (d, 1H), 5.63 (dd, 1H), 4.31 (m, 1H), 3.78 (m, 1H), 3.30 (m, 1H), 2.66 (m, 1H), 1.75 - 1.39 (m, 12H), 1.21 (d, 6H), 1.04 (m, 2H), OH protons fall in with $H_2O$.

Compound No. 28
IR (Neat); 3423.7 (broad), 2965 (s) 1728.4 (s). 1201 (broad) ($cm^{-1}$);
NMR ($CDCl_3$):
7.20 - 6.98 (m, 9H), 6.33 (d, 1H), 5.28 (g, 1H) 4.30 (m, 1H), 4.16 (m, 3H), 3.62 (s, 1H), 3.42 (m, 2H), 2.91 (s, 1H), 2.39 (dd, 2H), 1.34 (m, 6H), 1.28 (m, 3H);
HPLC : 84 : 16 (erythro : threo).

Compound No. 30
7.42 - 7.26 (m, 5), 6.58 (d, 1H), 5.72 (dd, 1H), 4.68 (m, 1H), 4.45 (m, 1H), 4.18 (q, 2H), 3.77 (d, 1H, OH), 3.43 (m, 1H), 3.27 (d, 1H, OH), 2.53 (d, 2H), 1.89 - 1.48 (m, 11 H), 1.30 (d, 6H), 1.29 (t, 3H),

Compound No. 31
NMR (DMSO-$d_6$)
7.47 - 7.26 (m, 5H), 6.42 (d, 1H), 5.68 (dd, 1H), 5.11 (d, 1H), 4.30 (m, 1H), 3.80 (m, 1H), 3.38 (m, 1H), 3.11 (m, 1H), 2.16 - 1.80 (m, 2H), 1.80 - 1.33 (m, 10H), 1.23 (d, 6H), other OH falls in with $H_2O$.

Compound No. 32
IR (Neat) : 3413.1 (broad), 1728.4 (s) 1245 (s), 2965 (s)
NMR (Neat): 7.24 (s, 1H), 7.08 (t, 6H), 6.80 (d, 2H), 6.30 (d, 1H), 5.40 (m, 1H), 4.36 (m, 1H) 4.18 (m, 3H), 3.89 (s, 3H), 3.62 (s, 1H), 3.46 (m, 2H), 2.82 (s, 1H), 2.44 (d, 2H), 1.62 (s, 1H), 1.34 (d, 6H), 1.28 (t, 3H).

Compound No. 33
7.12 (m, 10H), 6.24 (d, 1H), 5.29 (m, 1H), 4.08 (m, 1H), 3.80 (s, 3H), 3.46 (m, 1H), 2.44 (d, 2H), 1.58 (m, 2H), 1.40 (d, 6H).

Compound No. 34
7.56 - 7.01 (m, 9H), 6.36 (d, 1H), 5.29 (dd, 1H), 4.37 (m, 1H), 4.36 (q, 2H), 3.47 (m, 1H), 2.43 (d, 2H), 1.65 - 1.30 (m, 2H), 1.37 (d, 6H), 1.25 (t, 3H), OH protons not clear.

Compound No. 35
NMR (DMSO-$d_6$)
7.70 - 7.00 (m, 9H), 6.22 (d, 1H), 5.35 (dd, 1H), 4.11 (m, 1H), 3.58 (m, 1H), 3.45 (m, 1H), 2.09 - 1.70 (m, 2H), 1.50 -1.10 (m, 2H), 1.32 (d, 6H), OH protons fall in with $H_2O$.

Compound No. 37
NMR (DMSO-$d_6$)
7.75 - 7.03 (m, 14H), 6.21 (d, 1H), 5.44 (dd, 1H), 4.11 (m, 1H), 3.68 (m, 1H), 3.50 (m, 1H), 2.10 - 1.73 (m, 2H), 1.56 - 1.13 (m, 2H), 1.34 (d, 6H), OH protons fall in with $H_2O$.

Compound No. 38
Melting point 97-98°C.

Compound No. 39
7.08 (m, 4H), 6.23 (d, 1H), 5.18 (dd, 1H), 4.14 (m, 1H), 3.70 (m, 1H), 2.42 (d, 2H), 1.60 (m, 10H), 1.36 (d, 6H).

Compound No. 40
7.22 - 6.96 (m, 9H), 4.15 (m, 1H), 3.70 (q, 2H), 3.69 (m, 1H), 3.35 (m, 1H), 2.65 - 2.31 (m, 4H), 1.5 - 1.25 (m, 4H), 1.37 (d, 6H), 1.23 (t, 3H), OH protons not seen.

Compound No. 41
NMR (DMSO-$d_6$)
7.18 - 6.92 (m, 9H), 3.54 (m, 1H), 3.30 (m, 1H), 3.28 (m, 1H), 2.31 (m, 2H), 1.90 - 1.54 (m, 2H), 1.23 (d, 6H), 1.22 - 0.95 (m, 2H), OH protons fall in with $H_2O$.

Compound No. 42
Melting point 146-148°C.

Compound No. 43
6.95 (m, 5H), 6.38 (d, 1H), 5.22 (dd, 7H), 4.10 (m, 1H), 3.46 (m, 1H), 2.40 (d, 2H), 1.60 (m, 2H), 1.40 (dd, 12H).

**Claims**

1. Compounds of formula I

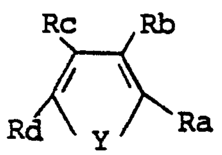

I

wherein
Ra is a group -X-Z, Rb is $R_2$, Rc is $R_3$, Rd is $R_4$ and Y is a group 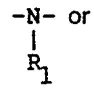 or

Ra is $R_1$, Rb is a group -X-Z, Rc is $R_2$, Rd is $R_3$ and Y is O, S or a group 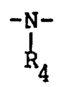 ;

$R_1$, $R_2$, $R_3$, and $R_4$ independently are $C_{1-6}$alkyl not containing an asymmetric carbon atom, $C_{3-7}$cycloalkyl or a ring

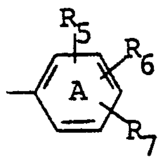

or in the case of $R_3$ and $R_4$ additionally hydrogen, or for $R_3$ when Y is O or S

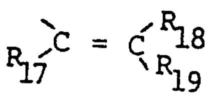

whereby $R_{17}$ is hydrogen or $C_{1-3}$alkyl and $R_{18}$ and $R_{19}$ are independently hydrogen $C_{1-3}$alkyl or phenyl; each $R_5$ is independently hydrogen, $C_{1-3}$alkyl, n-butyl, i-butyl, t-butyl, $C_{1-3}$alkoxy, n-butoxy, i-butoxy, trifluoromethyl, fluoro, chloro, bromo, phenyl, phenoxy or benzyloxy; each $R_6$ is independently hydrogen, $C_{1-3}$alkyl, $C_{1-3}$alkoxy, trifluoromethyl, fluoro, chloro, bromo, phenoxy or benzyloxy, and each $R_7$ is independently hydrogen, $C_{1-2}$alkyl, $C_{1-2}$alkoxy, fluoro or chloro, with the proviso that there may only be one each of trifluoromethyl, phenoxy or benzyloxy in each ring A present, X is $(CH_2)_m$ or $(CH_2)_qCH=CH-(CH_2)_q$, m is 0, 1, 2 or 3 and both q's are 0 or one is 0 and the other is 1,

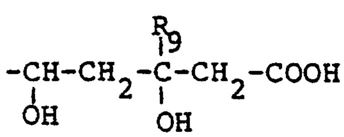

II

wherein $R_9$ is hydrogen or $C_{1-3}$alkyl,
in free acid form, or in the form of an ester or δ-lactone thereof or in salt form as appropriate.

2. Compounds according to Claim 1 wherein Ra is $R_1$, Rb is -X-Z, Rc is $R_2$, Rd is $R_3$ and Y is O or S, $R_1$, $R_2$ and $R_3$ are independently $C_{1-6}$alkyl not containing an asymmetric carbon atom, $C_{3-7}$cycloalkyl or a Ring B

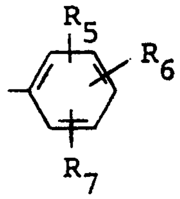

or in the case of $R_3$ additionally hydrogen, each $R_5$ is independently hydrogen, $C_{1-3}$alkyl, n-butyl, i-butyl, t-butyl, $C_{1-3}$alkoxy, n-butoxy, i-butoxy, trifluoromethyl, fluoro, chloro, phenyl, phenoxy or benzyloxy, each $R_6$ is independently hydrogen, $C_{1-3}$alkyl, $C_{1-3}$alkoxy, trifluoromethyl, fluoro, chloro, phenoxy or benzyloxy, and each $R_7$ is independently hydrogen, $C_{1-2}$alkyl, $C_{1-2}$alkoxy, fluoro or chloro with the proviso that there may only be one each of trifluoromethyl, phenoxy, or benzyloxy in each Ring B present, X is (E) CH = CH or $-CH_2-CH_2-$ and

Z is 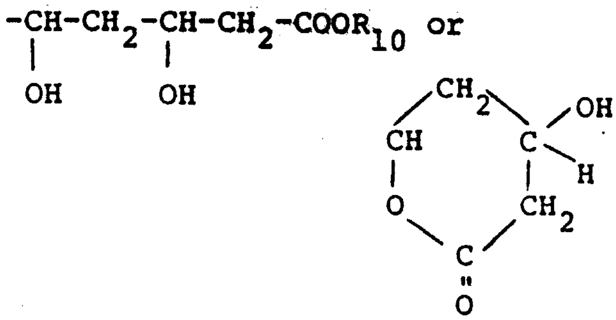 or

whereby $R_{10}$ is hydrogen, a physiologically acceptable ester group or a pharmaceutically acceptable cation.

3. Compounds according to Claim 1 wherein Ra is -X-Z, Rb is $R_2$, Rc is $R_3$, Rd is $R_4$ and Y is 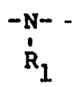 - and those wherein Ra is $R_1$, Rb is -X-Z, Rc is $R_2$, Rd is $R_3$ and Y is 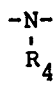 whereby $R_1$, $R_2$, $R_3$ and $R_4$ independently are $C_{1-6}$alkyl not containing an asymmetric carbon atom, $C_{3-7}$cycloalkyl or a Ring C

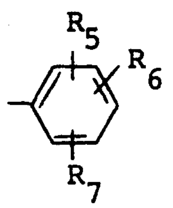

or in the case of $R_3$ and $R_4$ additionally hydrogen, each $R_5$ is independently hydrogen, $C_{1-3}$alkyl, n-butyl, ibutyl, t-butyl, $C_{1-3}$alkoxy, n-butoxy, i-butoxy, trifluoromethyl, fluoro, chloro, bromo, phenyl, phenoxy or benzyloxy, each $R_6$ is independently hydrogen, $C_{1-3}$alkyl, $C_{1-3}$alkoxy, trifluoromethyl, fluoro, chloro, phenoxy or benzyloxy, and each $R_7$ is independently hydrogen, $C_{1-2}$alkyl, $C_{1-2}$alkoxy, fluoro or chloro, with the proviso that there may only be one each of trifluoromethyl, phenoxy or benzyloxy in each Ring C present,

X is $(CH_2)_m$, -CH = CH-, CH = CH-CH$_2$ or CH$_2$CH = CH and Z is

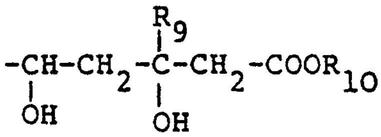

or

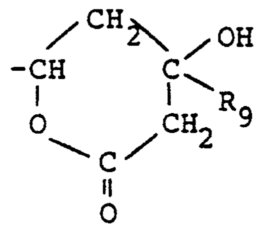

wherein $R_9$ is hydrogen or $C_{1-3}$alkyl and $R_{10}$ is hydrogen, a physiologically acceptable ester group or a pharmaceutically acceptable cation.

4. Compounds according to Claim 1 wherein the substituents are as defined hereinbefore in compound groups (i) to (ℓx) or Examples 1 to 12.

5. Sodium (±)-erythro-(E)-3,5-dihydroxy-7-[1'-(4"-fluorophenyl)-3'-(1"-methylethyl)-5'-phenyl-1H-pyrrol-2'-yl]hept-6-enoate.

6. A compound selected from Sodium (±)-erythro-(E)-7-[4-(4'-fluorophenyl)-2-isopropyl-5-phenylthien-3-yl]-3,5-dihydroxy-hept-6-enoate and Sodium (±)-erythro-(E)-7-[4-(4'-fluorophenyl-2,5-diisopropyl-thien-3-yl]-3,5-dihydroxy-hept-6-enoate.

7. A pharmaceutical composition comprising a compound according to Claim 1 as appropriate in free acid form or in the form of a physiologically-hydrolysable and -acceptable ester or a lactone thereof or in pharmaceutically acceptable salt form, together with a pharmaceutically acceptable diluent or carrier.

8. A compound according to Claim as appropriate in free acid form or in the form of a physiologically-hydrolysable and acceptable ester or a lactone thereof or in pharmaceutically acceptable salt form for use as a pharmaceutical.

9. A compound according to Claim 1 as appropriate in free acid form or in the form of a physiologically-hydrolysable and -acceptable ester or a lactone thereof or in pharmaceutically acceptable salt form for use as a hypolipoproteinemic or anti-atherosclerotic agent.

10. A process for preparing a compound according to Claim 1 which comprises

a) when $R_9$ is hydrogen reducing a compound of formula VIII

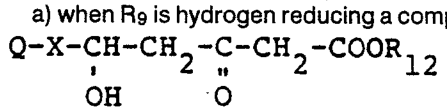 VIII

wherein $R_{12}$ is a radical forming an ester substantially inert to the reaction conditions and X is as defined above,

b) when $R_9$ is $C_{1-3}$alkyl hydrolysing a compound of formula XVI

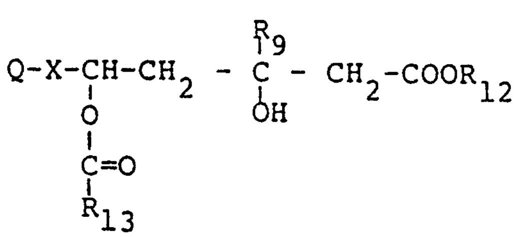 XVI

wherein $R_9$ is $C_{1-3}$alkyl, $R_{13}$ is part of an ester forming group and X and $R_{12}$ are as defined above,

c) when X is $-(CH_2)_2-$, $-(CH_2)_3-$, $-CH=CH-$ or $-CH_2CH=CH-$deprotecting a compound of formula XXVII

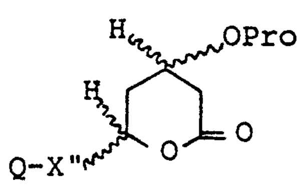 XXVII

wherein X" represents $-(CH_2)_2-$, $-(CH_2)_3$, $-CH=CH-$ or $-CH_2CH=CH-$

and Pro is a protecting group,

d) when X is $-(CH_2)_2-$, $-(CH_2)_3-$, or $-(CH_2)_qCH=CH(CH_2)_q$-deprotecting a compound of formula XI

$$Q-X'''-\underset{\displaystyle OPro}{\underset{|}{CH}}-CH_2-\underset{\displaystyle OPro}{\underset{|}{\overset{\displaystyle R_9}{\overset{|}{C}}}}-CH_2COOR_{12} \qquad XXII$$

wherein X"' is $-(CH_2)_2-$, $-(CH_2)_3-$ or $-(CH_2)_q-CH=CH-(CH_2)_q-$,
and q, $R_9$, $R_{12}$ and Pro are as defined above,

e) hydrolysing a compound of formula I in the form of an ester or a lactone,

f) esterifying or lactonising a compound of formula I in free acid form, or

g) esterifying a compound of formula I in lactone form, and when a free carboxyl group is present, recovering the compound obtained in free acid form or in the form of a salt.

11. A process for preparing a compound according to Claim 1 which comprises hydrolysing a compound of formula I in ester or lactone form or esterifying or lactonising a compound of formula I in free acid form or esterifying a compound of formula I inlactone form and when a free carboxyl group is present recovering the compound obtained in free acid form or in the form of a salt.

12. A compound of formulae VIII, XVI, XIX, XX, XXII, LXXIIa, XXIV, XXVII, XXVIIa, XXIX, XLV-XLVII, L-LII, LVIII, LX, LXIII, LXIV.

European Patent Office

# EUROPEAN SEARCH REPORT

Application number

EP 86810470.4

| Category | Citation of document with indication, where appropriate, of relevant passages | Relevant to claim | CLASSIFICATION OF THE APPLICATION (Int. Cl 4) |
|---|---|---|---|
| A | AT - B - 377 255 (RICHTER GEDEON VEGYÉSZETI GYÁR R.T.) * Claim 1; page 1 * -- | 1,7,12 | C 07 D 207/337<br>C 07 D 307/54<br>C 07 D 333/24<br>A 61 K 31/34<br>A 61 K 31/38<br>A 61 K 31/40 |
| A | DE - A - 2 261 965 (CONTINENTAL PHARMA) * Pages 1-3 * -- | 1,7,12 | |
| A | US - A - 3 168 532 (FRANKLIN WILLARD SHORT et al.) * Columns 1,2 * -- | 1,7,12 | |
| A | DE - A1 - 3 309 355 (BASF AG) * Claim 1 * -- | 1 | |
| | | | **TECHNICAL FIELDS SEARCHED (Int Cl 4)** |
| A | DE - A - 1 695 941 (THE UPJOHN CO.) * Pages 1,2 * -- | 1 | C 07 D 207/00<br>C 07 D 307/00<br>C 07 D 333/00<br>C 07 D 405/00<br>C 07 D 407/00<br>C 07 D 409/00<br>C 07 D 209/00<br>C 07 C 59/00 |
| A | EP - A1 - 0 155 524 (A. NATTERMANN & CIE. GMBH) * Claims * -- | 1,7,10, 12 | |
| A | US - A - 4 035 394 (S.S. PELOSI, JR. et al.) * Abstract; example II * -- | 1,7,10, 12 | |

The present search report has been drawn up for all claims

| Place of search | Date of completion of the search | Examiner |
|---|---|---|
| VIENNA | 16-01-1987 | HEIN |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

Application number: EP 86810470.4

**DOCUMENTS CONSIDERED TO BE RELEVANT**

| Category | Citation of document with indication, where appropriate, of relevant passages | Relevant to claim | CLASSIFICATION OF THE APPLICATION (Int. Cl.4) |
|---|---|---|---|
| D,A | EP - A1 - 0 114 027 (SANDOZ AG)<br>* Claims *<br>-- | 1,7,10,<br>12 | |
| D,A | EP - A1 - 0 117 228 (SANDOZ-PATENT-GMBH)<br>* Claims *<br>-- | 1,7,10, | |
| D,A | WO - A2/A3 - 86/00 307 (SANDOZ AG)<br>* Claims *<br>-- | 1,7,10,<br>12 | |
| D,A | WO - A1 - 86/03 488 (SANDOZ AG)<br>* Claims *<br>---- | 1,7,10,<br>12 | |

TECHNICAL FIELDS SEARCHED (Int. Cl.4)

The present search report has been drawn up for all claims

| Place of search | Date of completion of the search | Examiner |
|---|---|---|
| VIENNA | 16-01-1987 | HEIN |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82